# EUROPEAN PATENT APPLICATION

(11) **EP 4 450 518 A1**
(43) Date of publication of application: **23.10.2024**
(21) Application number: 22907488.5
(22) Date of filing: 14.12.2022
(51) Int. Cl.: C07K 16/12, A61K 38/08, A61K 39/395, A61P 31/04, A61P 43/00, C07K 16/46, C12N 15/13

(54) **VHH ANTIBODY**

(30) Priority: 15.12.2021 JP 2021203699
(71) Applicant: Teikyo Heisei University, Tokyo 170-8445 (JP); Juntendo Educational Foundation, Tokyo 113-8421 (JP)
(72) Inventor: OHNO, Maki, Tokyo 164-8530 (JP); ISHIDA, Isao, Tokyo 164-8530 (JP); KIRIKAE, Teruo, Tokyo 113-8421 (JP)
(74) Representative: Plougmann Vingtoft a/s
(86) International application number: PCT/JP2022/046069
(87) International publication number: WO 2023/112965

(57) **Abstract**

Provided is an excellent drug that increases the antimicrobial susceptibility of multidrug-resistant *Pseudomonas aeruginosa.* The present invention relates to a VHH antibody or a dimer or trimer thereof that recognizes loop 1 or loop 2 in OprM of an RND-type multidrug efflux pump of *Pseudomonas aeruginosa.*

## Description

### Technical Field

The present invention relates to an antibody that increases the antimicrobial susceptibility of multidrug-resistant *Pseudomonas aeruginosa.*

### Background Art

Multidrug-resistant gram-negative bacteria are main pathogenic bacteria of hospital infection, and appearance and spread of these bacteria are serious public health problems all over the world. The World Health Organization (WHO) announced in 2017 a list of drug-resistant pathogens with high urgency for new antimicrobial agent development, and carbapenem-resistant *Acinetobacter baumannii, Enterobacteriaceae,* and *Pseudomonas aeruginosa* were listed as pathogens in particularly critical phases. In Japan, infectious diseases caused by these pathogens are all designated as sentinel diseases under the Infectious Diseases Law, and medical facilities designated as a sentinel site are required to report the number of patients every month. Healthy adults rarely develop the infectious diseases caused by these pathogens, but for patients with immunodeficiency or serious underlying diseases, pneumonia and bacteremia caused by the bacterial infection are potentially fatal. It has been reported that in particular, the mortality rate of bacteremia caused by multidrug-resistant *Pseudomonas aeruginosa* (MDRP) is higher than that of other bacteria. According to the 2019 tally in the inpatient specimen testing department of Japan Nosocomial Infections Surveillance (JANIS), MDRP was isolated at 22.8% of the 2,075 target medical facilities.

MDRP is resistant to all of carbapenem, aminoglycoside, and fluoroquinolone antimicrobial agents which are generally effective against *Pseudomonas aeruginosa* infectious diseases. *Pseudomonas aeruginosa* acquires drug resistance through mechanisms such as expression of a drug efflux pump, production of β-lactamase, a decrease in permeability of an antimicrobial agent, and a mutation of DNA gyrase. In particular, the drug efflux pump uses a proton motive force to excrete various antimicrobial agents taken into a bacterial cell and causes multidrug resistance independently.

*Pseudomonas aeruginosa* is a gram-negative bacterium and has inner membrane, and outer membrane composed of a lipid bilayer and peptide glycan. Gram-negative bacteria express a complex called resistance-nodulation-cell division (RND) type multidrug efflux pump complex that penetrates both the inner membrane and the outer membrane, which excretes the drug taken into a bacterial cell to cause multidrug resistance. Twelve types of RND-type multidrug efflux pumps are known in *Pseudomonas aeruginosa.* In particular, MexAB-OprM has the highest expression level and excretes a wide range of drugs. MexAB-OprM is composed of OprM that penetrates the outer membrane and functions as a through-hole for drug efflux, MexB that is a transporter present in the inner membrane and performs drug efflux utilizing the proton concentration gradient, and MexA that connects between OprM and MexB. OprM forms a tubular channel in the outer membrane, and 2 types of loops (loop 1 and loop 2), three each, protrude to the outside the cell at the outlet for drug efflux. It has been reported that the minimum growth inhibitory concentration (MIC) of an antimicrobial agent decreased in an MexAB-OprM-deficient *Pseudomonas aeruginosa* strain, compared to the wild-type strain. There is a report that in also a *Pseudomonas aeruginosa* strain in which only OprM has been deleted, the MICs of norfloxacin and Aztreonam decreased to 1/7 and 1/15, respectively. Until now, companies and research institutes have developed efflux pump inhibitors, but no inhibitor that can be used clinically has been obtained yet. PaβN (phenyl-arginine-β-naphthylamide) is a competitive efflux pump inhibitor that is excreted by MexB of *Pseudomonas aeruginosa* and increases the concentration of an antimicrobial agent in the bacterial cell (Non Patent Literature 1), but the toxicity thereof was a problem (Non Patent Literatures 2 and 3). It has been reported that PaβN, 1-(1-naphthylmethyl)-piperazine (NMP), and phenothiazine decreased the MIC value of an antimicrobial agent in *Pseudomonas aeruginosa* to 1/8 or less, but none of them is used clinically as an efflux pump inhibitor (Non Patent Literatures 4 and 5).

OprM is a trimer, forms a tubular channel in the outer membrane, and 2 types of loops (loop 1 and loop 2) constituted of 10 amino acids, three each, protrude to the outside the cell at the drug outlet. Yoshihara et al, Tokai University, produced a mouse monoclonal antibody to loop 1 and loop 2 for the purpose of suppressing the function of drug efflux pump MexAB-OprM of *Pseudomonas aeruginosa* and increasing the susceptibility to antimicrobial agents, and reported that this antibody enhances the anti*-Pseudomonas aeruginosa* activity of Aztreonam (Patent Literature 1).

### Citation List

### Patent Literature

Patent Literature 1: WO 2007/091395

### Non Patent Literature

Non Patent Literature 1: J. Med. Chem., 1999, 42(24), 4928-4931
Non Patent Literature 2: Antimicrob. Agents Chemother., 2001, 45(1), 105-116
Non Patent Literature 3: J. Med., 2011, 6(1), 5870
Non Patent Literature 4: Antimicrob. Agents Chemother., 2008, 52(10), 3604-3611
Non Patent Literature 5: Antimicrob. Agents Chemother., 2014, 58(11), 6870-6878

### Summary of Invention

### Technical Problem

However, the mouse monoclonal antibody described in Patent Literature 1 enhances the anti-*Pseudomonas aeruginosa* activity of Aztreonam, but the effect is not sufficient, and development of a further excellent drug that increases the antimicrobial susceptibility of multidrug-resistant *Pseudomonas aeruginosa* is being desired.

Accordingly, an object of the present invention is to provide an excellent drug that increases the antimicrobial susceptibility of multidrug-resistant *Pseudomonas aeruginosa.*

### Solution to Problem

Accordingly, the present inventors produced a VHH antibody and a dimer and trimer thereof that recognize loop 1 or loop 2 in OprM of the RND-type multidrug efflux pump of *Pseudomonas aeruginosa* in order to develop a new antibody that increases the antimicrobial susceptibility of multidrug-resistant *Pseudomonas aeruginosa* and studied their functions. As a result, they found that this VHH antibody unexpectedly does not have an effect of increasing the susceptibility to antimicrobial agents such as Aztreonam to which the antibody described in Patent Literature 1 showed antimicrobial susceptibility, nevertheless, has an effect of significantly increasing the susceptibility to peptide antimicrobial agents such as colistin which are said to be a trump to multidrug-resistant *Pseudomonas aeruginosa,* and the effect mechanism is based on destabilization of the *Pseudomonas aeruginosa* outer membrane, and the present invention was accomplished.

That is, the present invention provides the following inventions [1] to [16]:
[1] A VHH antibody or a dimer or trimer thereof that recognizes loop 1 or loop 2 in OprM of an RND-type multidrug efflux pump of *Pseudomonas aeruginosa;*
[2] The VHH antibody or a dimer or trimer thereof according to [1], wherein the VHH antibody that recognizes the loop 1 has CDR1 having the amino acid sequence set forth in SEQ ID NO: 1 or an amino acid sequence having at least 90% homology to the amino acid sequence set forth in SEQ ID NO: 1, CDR2 having the amino acid sequence set forth in SEQ ID NO: 2 or an amino acid sequence having at least 90% homology to the amino acid sequence set forth in SEQ ID NO: 2, and CDR3 having the amino acid sequence set forth in SEQ ID NO: 3 or an amino acid sequence having at least 90% homology to the amino acid sequence set forth in SEQ ID NO: 3;
[3] The VHH antibody or a dimer or trimer thereof according to [1], wherein the VHH antibody that recognizes the loop 2 has CDR1 having the amino acid sequence set forth in SEQ ID NO: 5 or an amino acid sequence having at least 90% homology to the amino acid sequence set forth in SEQ ID NO: 5, CDR2 having the amino acid sequence set forth in SEQ ID NO: 6 or an amino acid sequence having at least 90% homology to the amino acid sequence set forth in SEQ ID NO: 6, and CDR3 having the amino acid sequence set forth in SEQ ID NO: 7 or an amino acid sequence having at least 90% homology to the amino acid sequence set forth in SEQ ID NO: 7;
[4] The VHH antibody or a dimer or trimer thereof according to [1] or [2], wherein the VHH antibody that recognizes the loop 1 has an amino acid sequence set forth in SEQ ID NO: 4 or an amino acid sequence having at least 90% homology to the amino acid sequence set forth in SEQ ID NO: 4;
[5] The VHH antibody or a dimer or trimer thereof according to [1] or [3], wherein the VHH antibody that recognizes the loop 2 has the amino acid sequence set forth in SEQ ID NO: 8 or an amino acid sequence having at least 90% homology to the amino acid sequence set forth in SEQ ID NO: 8;
[6] The VHH antibody or a dimer or trimer thereof according to any one of [1] to [5], wherein the antibody is a humanized antibody;
[7] The humanized VHH antibody or a dimer or trimer thereof according to [6], wherein the humanized VHH antibody that recognizes the loop 1 has the amino acid sequence set forth in SEQ ID NO: 9 or an amino acid sequence having at least 90% homology to the amino acid sequence set forth in SEQ ID NO: 9;
[8] The humanized VHH antibody or a dimer or trimer thereof according to [6], wherein the humanized VHH antibody that recognizes the loop 2 has the amino acid sequence set forth in SEQ ID NO: 10 or an amino acid sequence having at least 90% homology to the amino acid sequence set forth in SEQ ID NO: 10;
[9] A pharmaceutical composition comprising the VHH antibody or a dimer or trimer thereof according to any one of [1] to [8];
[10] The pharmaceutical composition according to [9], wherein the pharmaceutical composition increases susceptibility to a peptide antimicrobial agent in *Pseudomonas aeruginosa;*
[11] The pharmaceutical composition according to [9] or [10], wherein the pharmaceutical composition is used in combination with a peptide antimicrobial agent;
[12] The pharmaceutical composition according to [10] or [11], wherein the peptide antimicrobial agent is colistin or its salt;
[13] A *Pseudomonas aeruginosa* outer membrane-destabilizing agent comprising the VHH antibody or a dimer or trimer thereof according to any one of [1] to [8] as an active ingredient;
[14] The VHH antibody or a dimer or trimer thereof according to any one of [1] to [8], for use in increasing susceptibility to a peptide antimicrobial agent in *Pseudomonas aeruginosa;*
[15] Use of the VHH antibody or a dimer or trimer thereof according to any one of [1] to [8] for manufacturing a medicament for increasing susceptibility to a peptide antimicrobial agent in *Pseudomonas aeruginosa;* and
[16] A method for increasing susceptibility to a peptide antimicrobial agent in *Pseudomonas aeruginosa,* comprising administering the VHH antibody or a dimer or trimer thereof according to any one of [1] to [8].

### Advantageous Effects of Invention

The VHH antibody of the present invention has an effect of significantly increasing susceptibility to peptide antimicrobial agents such as colistin, which is said to be a potential trump card against multidrug-resistant *Pseudomonas aeruginosa,* and the effect mechanism is based on destabilization of the *Pseudomonas aeruginosa* outer membrane. The effect of increasing the susceptibility to peptide antimicrobial agents by the VHH antibody of the present invention is also confirmed in vivo, and the dose of peptide antimicrobial agents such as colistin can also be decreased.

### Brief Description of Drawings

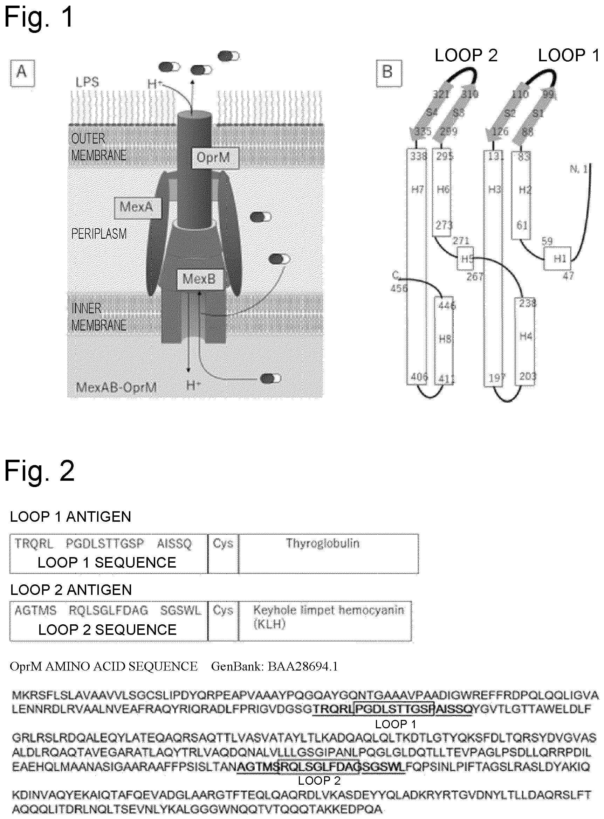

Fig. 1 shows the structure of MexAB-OprM and the secondary structure of OprM. A: A schematic diagram illustrating the structure of MexAB-OprM, MexAB-OprM is the RND-type multidrug efflux pump of *Pseudomonas aeruginosa* and is composed of OprM that penetrates the outer membrane and functions as a through-hole for drug efflux, MexB that is a transporter present in the inner membrane and performs drug efflux by using a proton concentration gradient, and MexA that connects between OprM and MexB. B: A secondary structure of OprM, OprM is a large domain consisting of α-helix and interacts with MexA and MexB, and β-sheets come together to form a barrel-shaped structure in the domain penetrating the outer membrane. Two types of loops (loop 1 and loop 2) protruding to the outside the cell, and each thereof consists of 10 amino acids.

Fig. 2 shows the amino acid sequence of OprM and the designs of antigens. Peptides that are each composed of 10 amino acids of the extracellularly protruding loop 1 or loop 2 of OprM, sequences of 5 amino acids added to before and after the 10 amino acids, and cysteine added to the C-terminal thereof were synthesized, and thyroglobulin and Keyhole limpet hemocyanin (KLH) were added as carrier proteins to the loop 1 peptide and the loop 2 peptide, respectively, to obtain antigens. The amino acid sequence of OprM is shown in SEQ ID NO: 15.

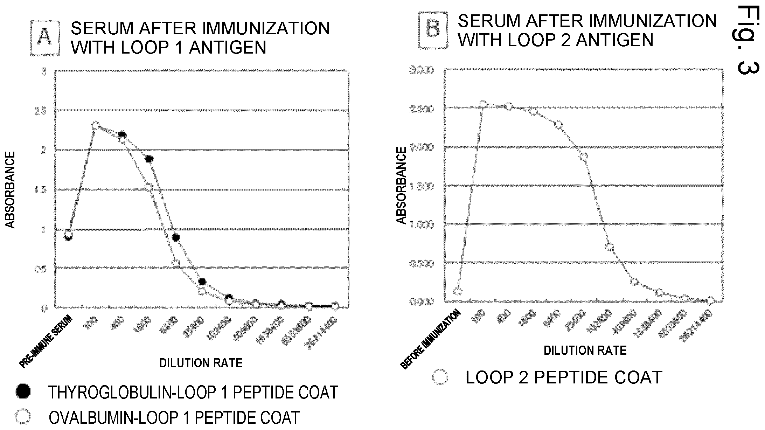

Fig. 3 shows the results of antibody titer measurement of alpaca serum. ELISA was performed using serum collected 5 weeks after immunization with the loop 1 antigen or serum collected 7 weeks after immunization with the loop 2 antigen. A: Serum after immunization with the loop 1 antigen was reacted with immobilized thyroglobulin-loop 1 peptide or ovalbumin-loop 1 peptide, and the bound antibody was detected with an anti-llama IgG-HRP. B: Serum after immunization with the loop 2 antigen was reacted with immobilized loop 2 peptide, and the bound antibody was detected with an anti-llama IgG-HRP.

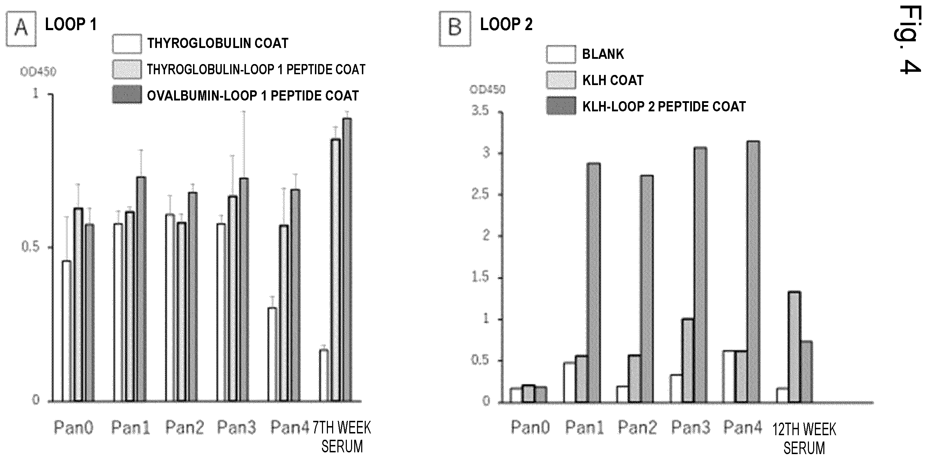

Fig. 4 shows the results of enrichment of specific antibodies against antigens by biopanning. Before panning, ELISA was performed using phage libraries before panning and after the 1st to 4th rounds of panning. A: The immobilized antigens used were thyroglobulin, thyroglobulin-loop 1 peptide, and ovalbumin-loop 1 peptide, and detection was performed with an anti-M13 monoclonal antibody and an anti-mouse IgG-HRP. B: The immobilized antigens used were NaHCO₃ (blank), KLH, and KLH-loop 2 peptide, and detection was performed with an anti-M13 monoclonal antibody and an anti-mouse IgG-HRP.

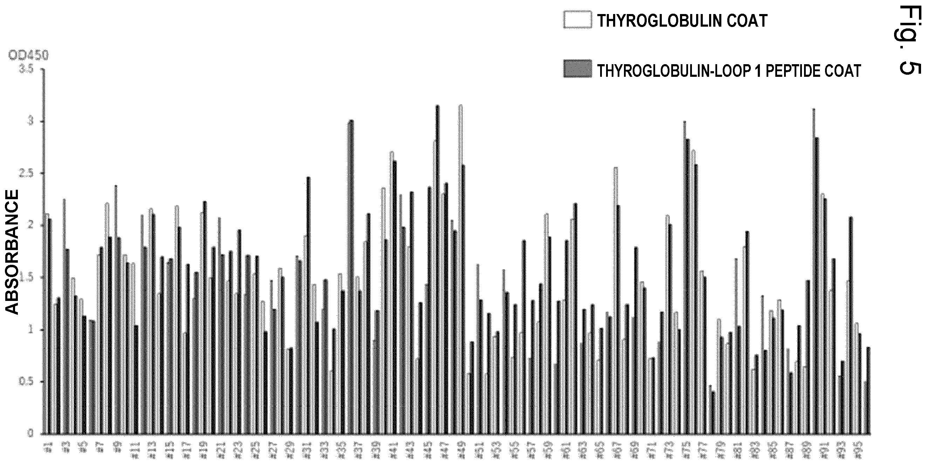

Fig. 5 shows the results of screening for loop 1 antigen-specific binding phages. 96 phages were cloned from the loop 1 library after the 4th round of panning, and ELISA was performed. The immobilized antigens used were thyroglobulin and thyroglobulin-loop 1 peptide, and detection was performed with an anti-M13 monoclonal antibody and an anti-mouse IgG-HRP.

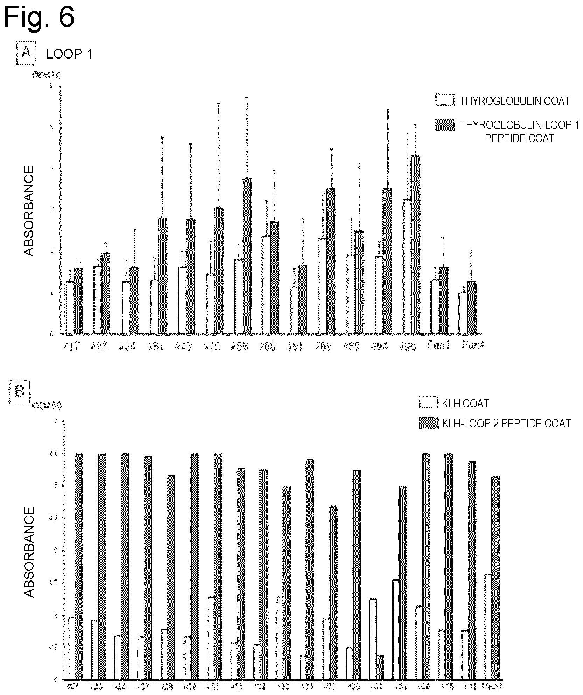

Fig. 6 shows the results of ELISA of isolated phage clones. A: ELISA of monoclonal phages was performed after biopanning against the loop 1 antigen 4 times. The immobilized antigens used were thyroglobulin and thyroglobulin-loop 1 peptide, and detection was performed with an anti-M13 monoclonal antibody and an anti-mouse IgG-HRP. B: ELISA of monoclonal phages was performed after biopanning against the loop 2 antigen 4 times. The immobilized antigens used were KLH and KLH-loop 2 peptide, and detection was performed with an anti-M13 monoclonal antibody and an anti-mouse IgG-HRP.

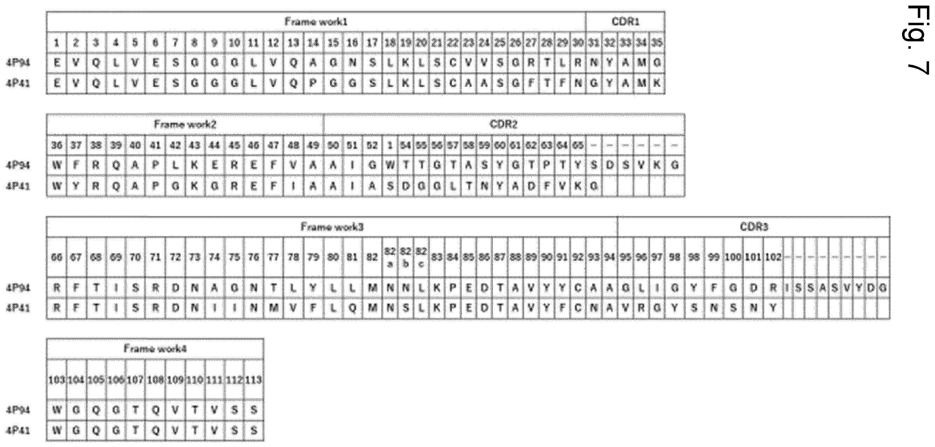

Fig. 7 shows amino acid sequences of VHH specifically bound to antigens: amino acid sequences of 4P94 that is VHH specifically bound to the loop 1 antigen and 4P41 that is VHH specifically bound to the loop 2 antigen.

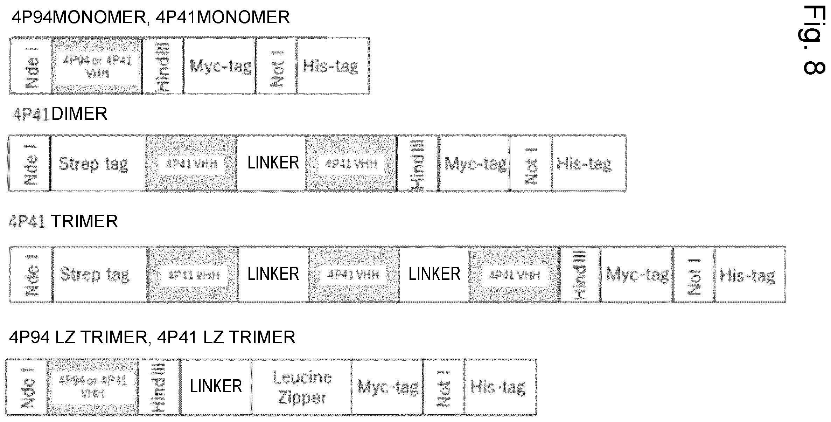

Fig. 8 shows the structures of antibodies. Genes for *Escherichia coli* expression of a 4P94 monomer, a 4P94 LZ trimer, a 4P41 monomer, a 4P41 dimer, a 4P41 trimer, and a 4P41 LZ trimer were designed based on the amino acid sequence information on 4P94 and 4P41. As the trimers, an antibody in which three VHHs were linked in series and an antibody in which three VHHs were assembled via a leucine zipper were produced.

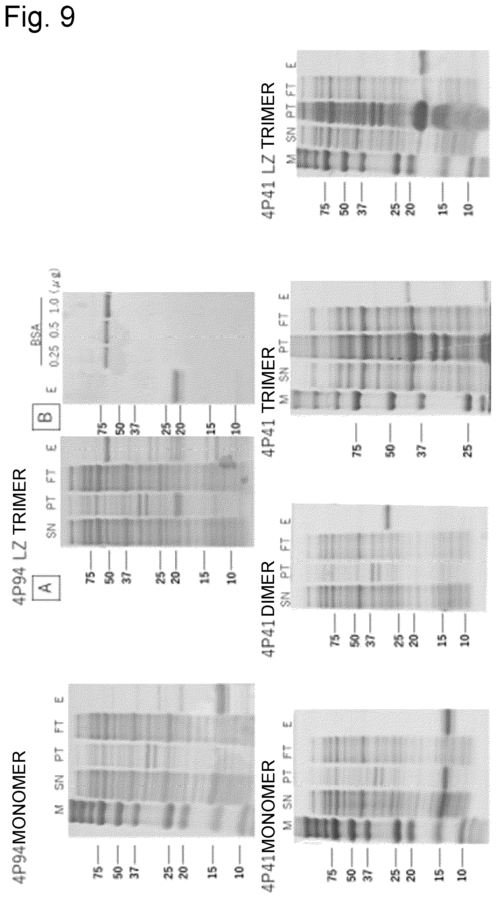

Fig. 9 shows the results of SDS-PAGE of purified antibodies. Genes for *Escherichia coli* expression of a 4P94 monomer, a 4P94 LZ trimer, a 4P41 monomer, a 4P41 dimer, a 4P41 trimer, and a 4P41 LZ trimer were DNA-synthesized and were introduced into *Escherichia coli* RosettaGami^{™} 2 (DE3) (Novagen). Expression was induced by addition of IPTG, the prepared soluble fraction was column-purified described in the method, and SDS-PAGE was performed. The 4P94 LZ trimer was eluted using a His-Trap column (A), and the eluted sample was purified by ion exchange chromatography (B). M: marker, SN: soluble fraction, PT: precipitation fraction, FT: flow-through fraction, E: eluted fraction.

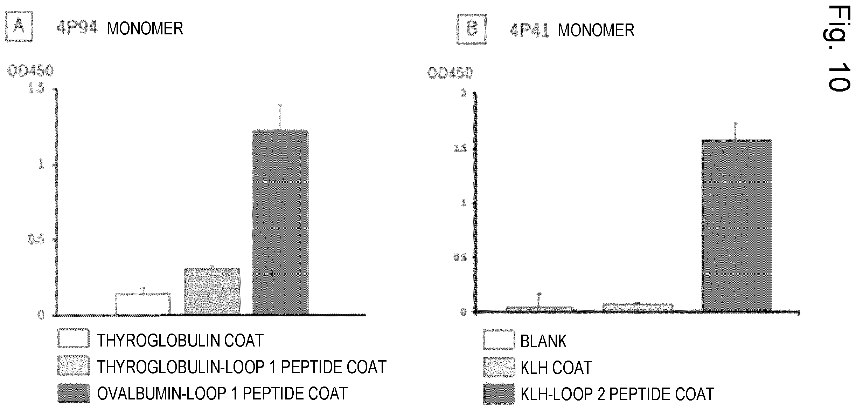

Fig. 10 shows the avidity of the 4P94 monomer and 4P41 monomer to the antigen. A: The avidity of the 4P94 monomer to the loop 1 antigen was measured by ELISA. The immobilized antigens used were thyroglobulin, thyroglobulin-loop 1 peptide, and ovalbumin-loop 1 peptide, and detection was performed with an anti-M13 monoclonal antibody and an anti-mouse IgG-HRP. B: The avidity of the 4P41 monomer to the loop 2 antigen was measured by ELISA. The immobilized antigens used were PBS, KLH, and KLH-loop 2 peptide, and detection was performed with an anti-M13 monoclonal antibody and an anti-mouse IgG-HRP.

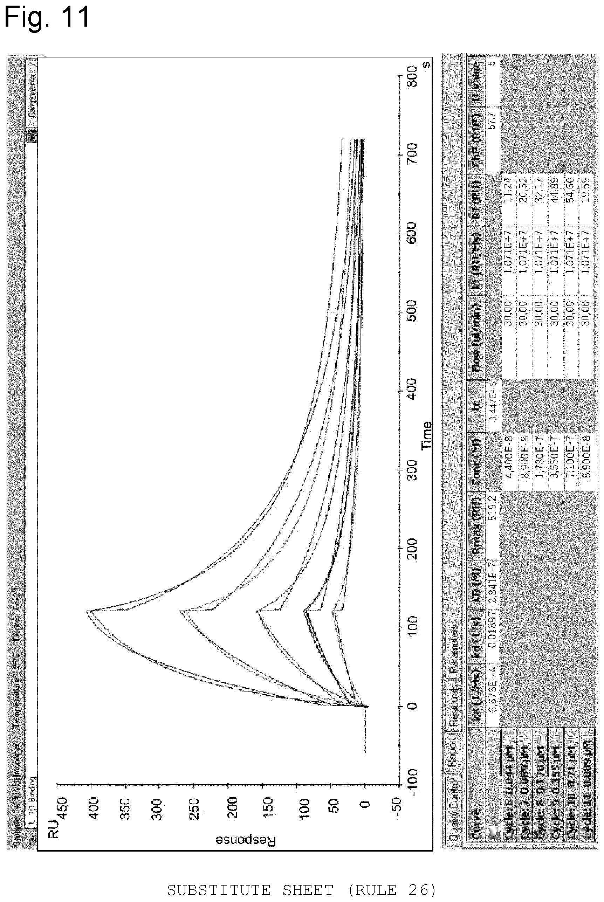

Fig. 11 shows the binding affinity between the 4P41 monomer and the loop 2 antigen. The binding affinity between the 4P41 monomer and the KLH-loop 2 was analyzed by surface plasmon resonance using Biacore X-100 (Cytiva). A multicycle kinetics method was performed to measure the 4P41 monomer of 4.4 nM, 89 nM, 178 nM, 355 nM, and 710 nM.

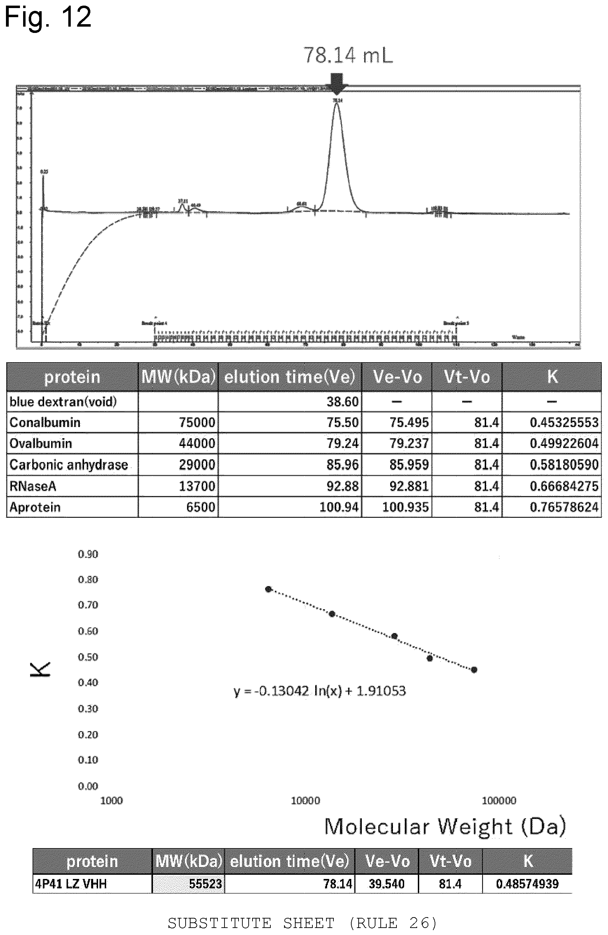

Fig. 12 shows the result of gel filtration chromatography of the 4P41 LZ trimer. A purified 4P41 LZ trimer sample was analyzed with a gel filtration column HiLoad16/60 Superdex 200 pg. The molecular weight was estimated from a calibration curve using globular proteins of known molecular weights as an indicator.

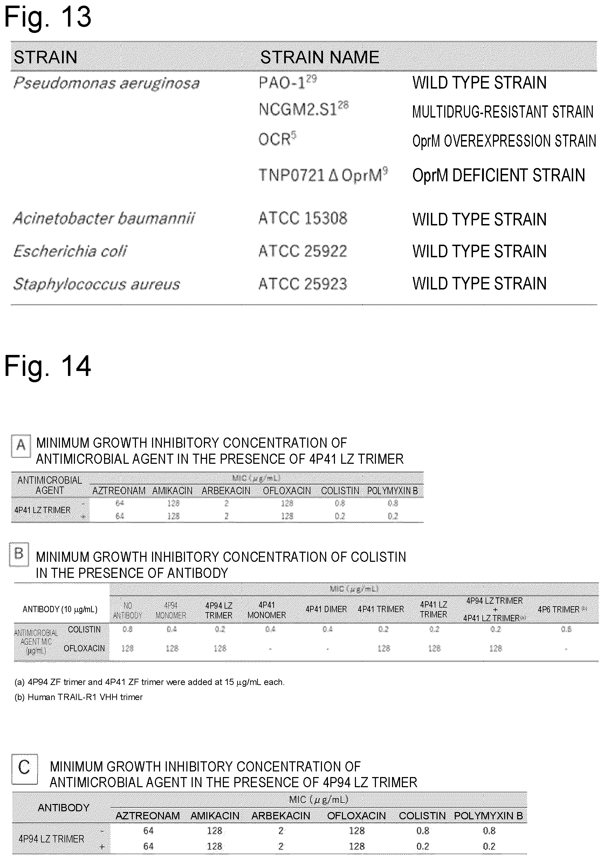

Fig. 13 shows strains used. The strains used in experiment are those of *Pseudomonas aeruginosa, Acinetobacter baumannii, Escherichia coli,* and *Staphylococcus aureus.*

Fig. 14 shows influences of 4P94 and 4P41 on antimicrobial agents. A: Minimum growth inhibitory concentrations (MICs) of antimicrobial agents in the presence of the 4P41 LZ trimer. The MIC of each antimicrobial agent in the presence of the 4P41 LZ trimer in NCGM2.S1 was measured by a microbroth dilution method in accordance with the CLSI guideline. B: MIC of colistin in the presence of an antibody. The MIC of colistin in the presence of each antibody in NCGM2.S1 was measured by a microbroth dilution method in accordance with the CLSI guideline. The antibodies were added at 10 µg/mL each, and in 4P94 LZ trimer + 4P41 LZ trimer, both were added at 15 µg/mL each. As the negative control, a 4P6 trimer which is a human TRAIL-R1 VHH trimer was used. C: Minimum growth inhibitory concentrations (MICs) of antimicrobial agents in the presence of the 4P94 LZ trimer. The MIC of each antimicrobial agent in the presence of the 4P94 LZ trimer in NCGM2.S1 was measured by a microbroth dilution method in accordance with the CLSI guideline.

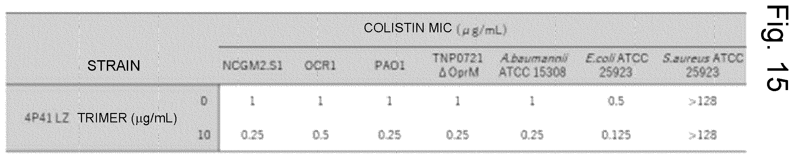

Fig. 15 shows the colistin susceptibility of each bacterium in the presence of the 4P41 LZ trimer. The MICs of colistin to *Pseudomonas aeruginosa* NCGM2.S1, OCR1, PAO1, and TNP0721ΔOprM, *Acinetobacter baumannii* ATCC15308, *Escherichia coli* ATCC25922, and *Staphylococcus aureus* ATCC25923 were measured in the absence of the 4P41 LZ trimer and in the presence of 10 µg/mL of the 4P41 LZ trimer by the microbroth dilution method in accordance with the CLSI guideline.

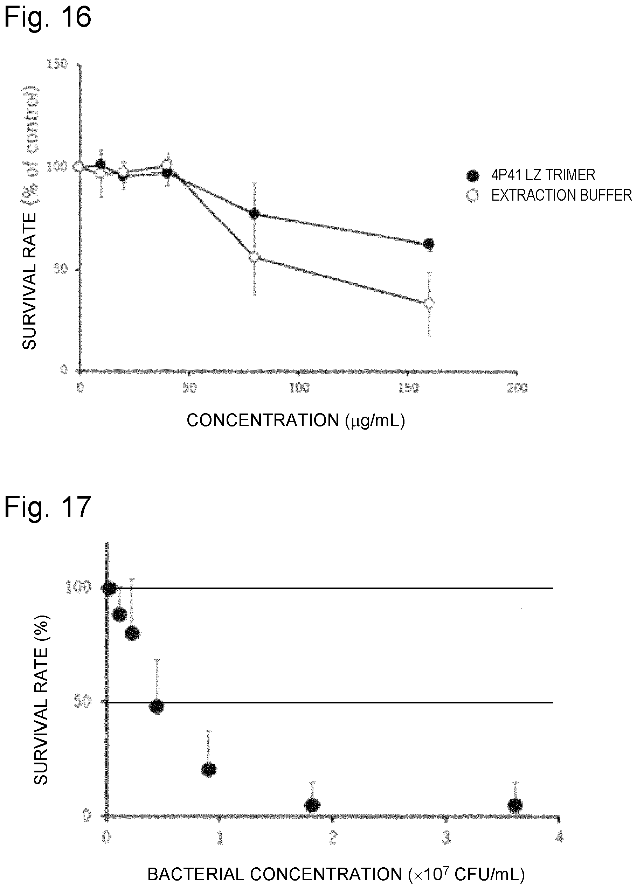

Fig. 16 shows the cytotoxicity of the 4P41 LZ trimer in HepG2. The 4P41 LZ trimer and an extraction buffer (50 mM Na phosphate, pH 7.8, 300 mM NaCl) as a solvent were added to human liver cancer HepG2 cells, and after culturing for 48 hours, Cell Counting Kit-8 was added thereto, followed by measurement of absorbance at 450 nm. The relative values to the value of the cells alone defined as 100% were used as data, which were shown by average value of 5 wells at each concentration + standard error.

Fig. 17 shows the NCGM2.S1 administration concentrations and survival rates in silkworm. Overnight culture solution of NCGM2.S1 was diluted by 100 times and cultured for 16 hours and was then injected into hemolymph of silkworms on the 2nd day of the 5th instar, and the survival rate after 24 hours was measured.

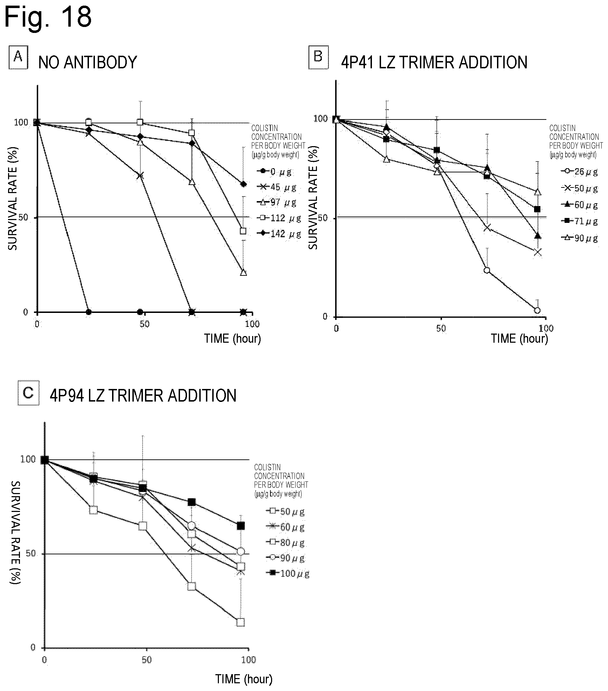

Fig. 18 shows the therapeutically effective dose of colistin in a silkworm NCGM2.S1 infection model. A: Overnight culture solution of NCGM2.S1 was diluted by 100 times and cultured for 16 hours and was then injected into hemolymph of silkworms on the 2nd day of the 5th instar, and 0.05 mL of colistin was then injected into the blood. The survival rates were measured over 96 hours. B: Overnight culture solution of NCGM2.S1 was diluted by 100 times and cultured for 16 hours and was then injected into hemolymph of silkworms on the 2nd day of the 5th instar, and 0.05 mL of a solution mixture of colistin and the 4P41 LZ trimer was then injected into hemolymph. The survival rates were measured over 96 hours. C: Overnight culture solution of NCGM2.S1 was diluted by 100 times and cultured for 16 hours and was then injected into hemolymph of silkworms on the 2nd day of the 5th instar, and 0.05 mL of a solution mixture of colistin and the 4P94 LZ trimer was then injected into hemolymph. The survival rates were measured over 96 hours.

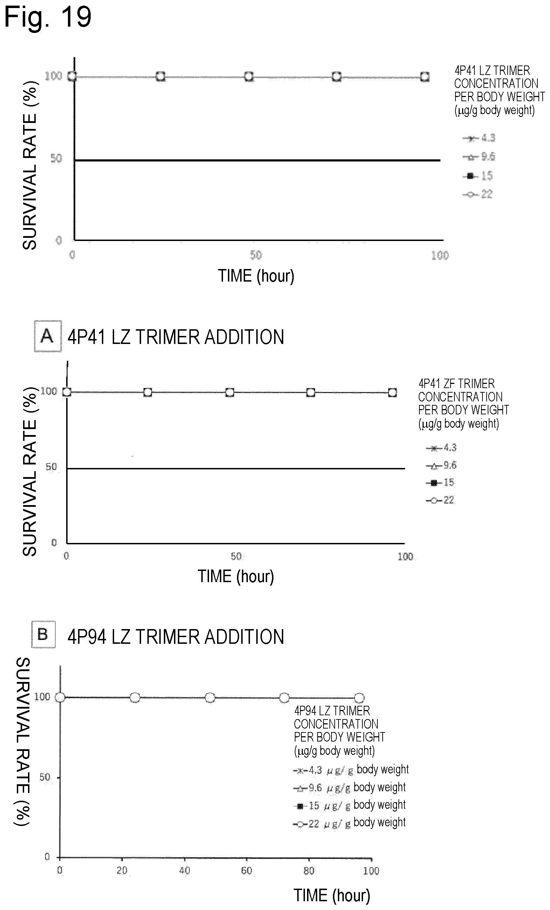

Fig. 19 shows the results of a toxicity test of 4P41 LZ trimer and 4P94 LZ trimer in silkworms. The 4P41 LZ trimer (A) or the 4P94 LZ trimer (B) was injected at each concentration into hemolymph of silkworms on the 2nd day of the 5th instar, and the survival rates were measured over 96 hours.

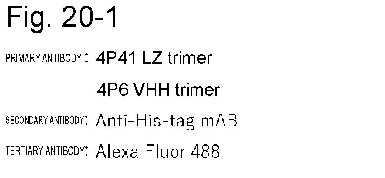

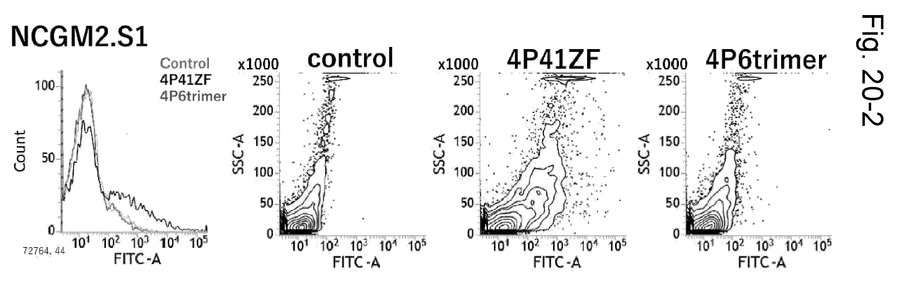

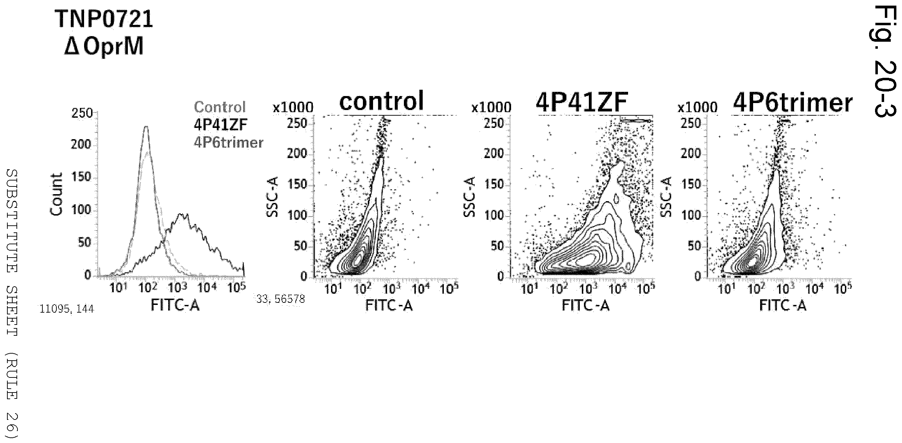

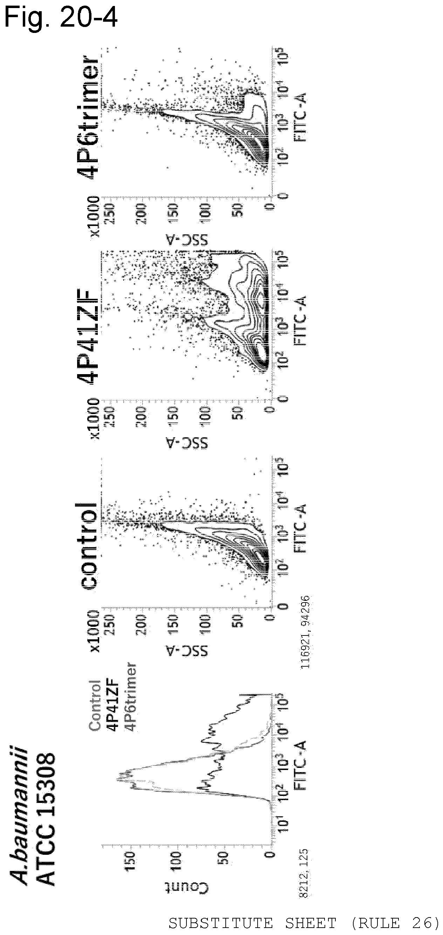

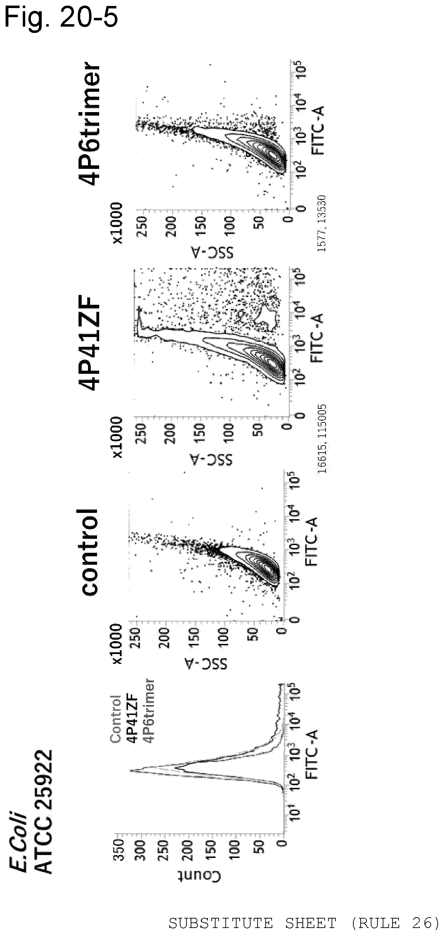

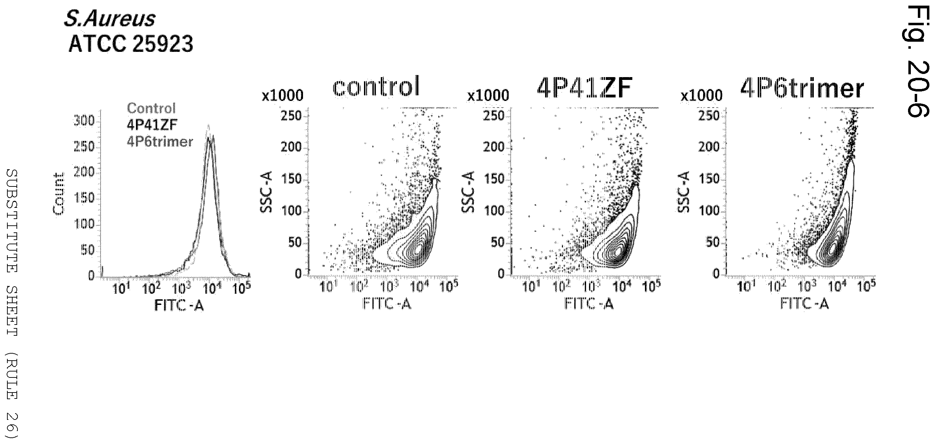

Fig. 20 shows the results of FACS analysis of the 4P41 LZ trimer and each bacterium. The binding between an antibody and bacteria was investigated by FACS analysis using 1 × 10⁸ CFU/mL of each bacterium, the 4P41 LZ trimer as a primary antibody or the 4P6 VHH trimer as a negative control, Anti-His-tag mA as a secondary antibody, and Alexa Fluor^{™} 488-conjugated AffiniPure Goat Anti-Mouse IgG as a tertiary antibody.

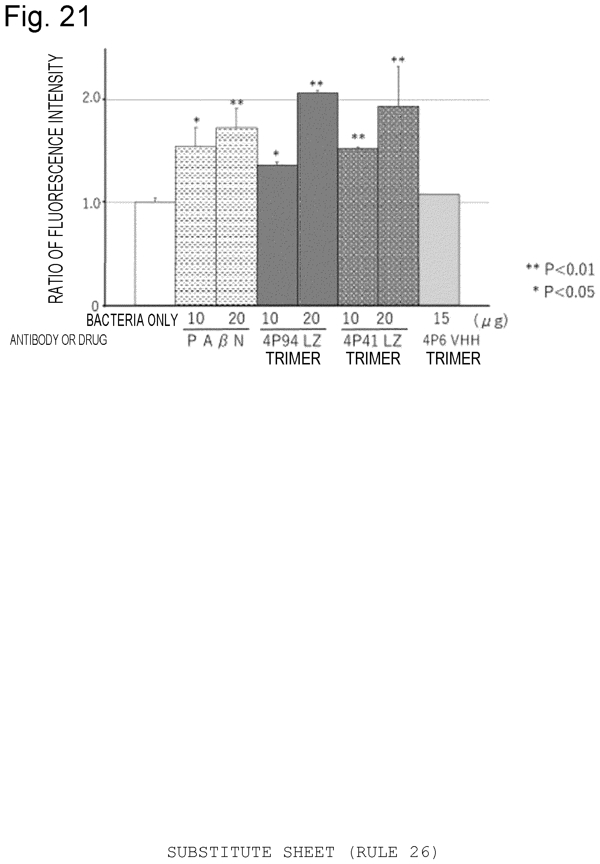

Fig. 21 shows influences of antibodies on the membrane of *Pseudomonas aeruginosa.* Influences of antibodies on the membrane of NCGM2.S1 were investigated using DiBAC₄. DiBAC4 was added to NCGM2.S1, and fluorescence intensity when PaβN as a pump inhibitor, the 4P94 LZ trimer, the 4P41 LZ trimer, or the 4P6 VHH trimer as an anti-human TRAIL-R2 VHH antibody was added thereto was measured. The fluorescence intensity ratios are shown with the fluorescence intensity of the group to which no antibody was added as 1.

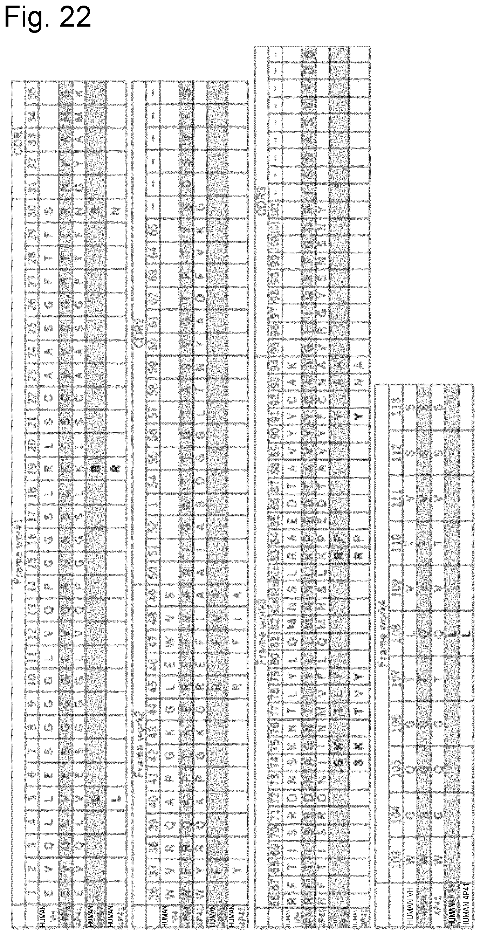

Fig. 22 shows humanized 4P94 and humanized 4P41 amino acid sequences. The framework sequences of 4P94 and 4P41 were humanized. The regions considered for humanization are noted in human 4P94 and human 4P41, and positions where the sequence differs from the original antibody due to humanization are shown in bold.

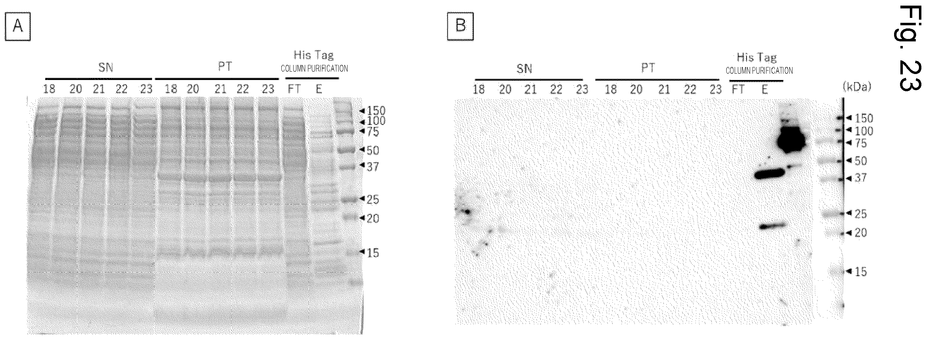

Fig. 23 shows the results of SDS-PAGE of purified antibodies and the results of western blotting using an anti-His-Tag mouse monoclonal antibody HRP. A gene for *Escherichia coli* expression of a humanized 4P41 LZ trimer was DNA-synthesized and was introduced into *E. coli* RosettaGami^{™} 2 (DE3) (Novagen). Expression was induced by addition of IPTG, and the prepared soluble fraction was purified with a His-Trap column (Cytiva), and SDS-PAGE was then performed (A). Western blotting using an anti-His-Tag mouse monoclonal antibody HRP (Thermo Fisher Scientific) was performed (B). M: marker, SN: soluble fraction, PT: precipitation fraction, FT: flow-through fraction, E: eluted fraction.

### Description of Embodiments

The present invention is a VHH antibody or a dimer or trimer thereof that recognizes loop 1 or loop 2 in OprM of an RND-type multidrug efflux pump of *Pseudomonas aeruginosa.*

Among RND-type multidrug efflux pumps of *Pseudomonas aeruginosa,* OprM is a drug efflux pump that has the highest expression level and excretes a wide range of drugs. OprM is a trimer, forms a tubular channel in the outer membrane, and 2 types of loops (loop 1 and loop 2) each constituted of 10 amino acids, three each, protrude to the outside the cell at the drug outlet (see Fig. 1).

The VHH antibody of the present invention includes a VHH antibody that recognizes loop 1 and a VHH antibody that recognizes loop 2.

In the VHH antibody that recognizes the loop 1, preferably, CDR1 has an amino acid sequence set forth in SEQ ID NO: 1 or an amino acid sequence having at least 90% homology to the amino acid sequence set forth in SEQ ID NO: 1, CDR2 has an amino acid sequence set forth in SEQ ID NO: 2 or an amino acid sequence having at least 90% homology to the amino acid sequence set forth in SEQ ID NO: 2, and CDR3 has an amino acid sequence set forth in SEQ ID NO: 3 or an amino acid sequence having at least 90% homology to the amino acid sequence set forth in SEQ ID NO: 3.

Here, the term homology means the percentage identity of amino acid sequences. The homology of these amino acid sequences is preferably 95% or more, more preferably 96% or more, further more preferably 98% or more, and even more preferably 99% or more.

The VHH antibody that recognizes the loop 1 preferably has an amino acid sequence set forth in SEQ ID NO: 4 or an amino acid sequence having at least 90% homology to the amino acid sequence set forth in SEQ ID NO: 4.

The homology of these amino acid sequences is preferably 95% or more, more preferably 96% or more, further more preferably 98% or more, and even more preferably 99% or more.

As the VHH antibody that recognizes the loop 2, preferably, CDR1 has an amino acid sequence set forth in SEQ ID NO: 5 or an amino acid sequence having at least 90% homology to the amino acid sequence set forth in SEQ ID NO: 5, CDR2 has an amino acid sequence set forth in SEQ ID NO: 6 or an amino acid sequence having at least 90% homology to the amino acid sequence set forth in SEQ ID NO: 6, and CDR3 has an amino acid sequence set forth in SEQ ID NO: 7 or an amino acid sequence having at least 90% homology to the amino acid sequence set forth in SEQ ID NO: 7.

The homology of these amino acid sequences is preferably 95% or more, more preferably 96% or more, further more preferably 98% or more, and even more preferably 99% or more.

The VHH antibody that recognizes the loop 2 preferably has an amino acid sequence set forth in SEQ ID NO: 8 or an amino acid sequence having at least 90% homology to the amino acid sequence set forth in SEQ ID NO: 8.

The homology of these amino acid sequences is preferably 95% or more, more preferably 96% or more, further more preferably 98% or more, and even more preferably 99% or more.

The VHH antibody of the present invention can be produced by immunizing a Camelidae animal with a peptide including an amino acid sequence of a loop 1 region or a peptide including an amino acid sequence of a loop 2 region, and the obtained VHH antibody can be humanized.

The humanized VHH antibody that recognizes the loop 1 preferably has an amino acid sequence set forth in SEQ ID NO: 9 or an amino acid sequence having at least 90% homology to the amino acid sequence set forth in SEQ ID NO: 9. The homology of these amino acid sequences is preferably 95% or more, more preferably 96% or more, further more preferably 98% or more, and even more preferably 99% or more.

The humanized VHH antibody that recognizes the loop 2 preferably has an amino acid sequence set forth in SEQ ID NO: 10 or an amino acid sequence having at least 90% homology to the amino acid sequence set forth in SEQ ID NO: 10. The homology of these amino acid sequences is preferably 95% or more, more preferably 96% or more, further more preferably 98% or more, and even more preferably 99% or more.

The antibody of the present invention is a VHH antibody, i.e., a single chain heavy chain small molecular antibody, and includes a monomer, a dimer, and a trimer. Among them, from the viewpoint of the effect of increasing the susceptibility to peptide antimicrobial agents, a trimer is particularly preferable.

The VHH antibody of the present invention can be produced by immunizing a Camelidae animal with a peptide including an amino acid sequence of the extracellularly protruding loop 1 region of OprM or a peptide including an amino acid sequence of loop 2 region, but since the VHH antibody is highly expressed in bacteria such as *Escherichia coli,* the VHH antibody can be manufactured by designing a monomer, dimer, or trimer of the VHH antibody, artificially synthesizing a VHH antibody gene, and performing expression in a recombinant *Escherichia coli* and purification.

As the Camelidae animal, alpaca can be used. As the antigen, a peptide including an amino acid sequence of a part of loop 1 or loop 2 can be used, and a peptide in which a carrier protein is added to a peptide including 10 to 20 amino acid residues is preferably used. As the carrier protein, for example, thyroglobulin or Keyhole limpet hemocyanin (KLH) can be used.

Monocytes are prepared from the blood obtained by immunization of a Camelidae animal, and RNA extraction from the monocyte and cDNA synthesis are performed. The gene of a VHH region is amplified from the obtained cDNA by PCR, and the VHH gene is introduced into *Escherichia coli* or the like to obtain a phage library. Cloned phages displaying VHH antibodies are obtained from the produced phage library by panning, clones that react with a loop 1 or loop 2 antigen are selected from the phages, and sequences of antibody genes can be determined.

Furthermore, once the sequence of an antibody gene has been determined, the VHH antibody can be manufactured by designing a monomer, dimer, or trimer of the VHH antibody, artificially synthesizing a VHH antibody gene, and performing expression in a recombinant *Escherichia coli* and purification.

The VHH antibody of the present invention can also be humanized. The humanized VHH antibody of the present invention is obtained by, for example, designing a DNA sequence such that the CDR of an alpaca VHH antibody is linked to the framework region (FR) of a human antibody, synthesizing the DNA sequence by PCR from several oligonucleotides produced so as to have an overlapping portion at the end, inserting the obtained DNA into a vector, and introducing the vector into a host such as *Escherichia coli* for expression.

The VHH antibody of the present invention showed no function of decreasing the MIC (minimum bacterial growth inhibitory concentration) of various antimicrobial agents (new quinolones, penicillins, carbapenems, aminoglycosides, and macrolides) including Aztreonam. However, in vitro activity of enhancing the antibacterial activity of peptide antimicrobial agents (colistin and polymyxin) was recognized. In addition, a silkworm model of *Pseudomonas aeruginosa* infection was produced, and the in vivo effectiveness of the VHH antibody of the present invention was investigated. As a result, the activity of enhancing the antibacterial activity of colistin was also recognized in vivo. Furthermore, the influence of the VHH antibody of the present invention on the bacterial outer membrane was investigated using a membrane potential-sensitive dye, and it was suggested that the potential of the *Pseudomonas aeruginosa* outer membrane changes antibody dose-dependently and that the effect of colistin is enhanced by destabilizing the *Pseudomonas aeruginosa* outer membrane by the VHH antibody of the present invention.

Accordingly, the VHH antibody or a dimer or trimer thereof of the present invention is useful as an active ingredient of a pharmaceutical composition, in particular, a pharmaceutical composition that increases the peptide antimicrobial susceptibility of *Pseudomonas aeruginosa.* The VHH antibody or a dimer or trimer thereof of the present invention is also useful as a *Pseudomonas aeruginosa* outer membrane-destabilizing agent.

A pharmaceutical composition containing the VHH antibody or a dimer or trimer thereof of the present invention can be formulated into a preparation for oral administration or for parenteral administration by, for example, mixing, dissolving, emulsifying, capsulating, or lyophilizing the VHH antibody or a dimer or trimer thereof of the present invention together with a pharmaceutically acceptable carrier.

Examples of the preparation suitable for oral administration include a liquid in which an effective dose of the present inventive VHH antibody is dissolved in water or a diluent such as physiological saline; a capsule, granule, powder, or tablet containing an effective dose of the inventive VHH antibody as a solid or granule; a suspension in which an effective dose of the inventive VHH antibody is suspended in an appropriate dispersion medium; and an emulsion obtained by dispersing and emulsifying a solution in which an effective dose of the inventive VHH antibody is dissolved in an appropriate dispersion medium.

In the preparation for parenteral administration, the present inventive VHH antibody can be formulated together with a pharmaceutically acceptable solvent, excipient, binder, stabilizer, dispersant, or the like into a dosage form such as injectable solution, suspension, emulsion, cream, ointment, inhalant, and suppository. In a prescription for injection, the present inventive VHH antibody can be dissolved in an aqueous solution, preferably in a physiologically compatible buffer such as Hanks' solution, Ringel's solution, or a physiological saline buffer. Furthermore, the drug of the present invention can take a form of suspension, solution, emulsion, or the like in an oilbased or water-based vehicle. Alternatively, the present inventive VHH antibody may be produced in a powder form and may be prepared into an aqueous solution or suspension using sterile water or the like before use. For administration by inhalation, the present inventive VHH antibody is powdered and can be formed into a powder mixture with an appropriate base such as lactose or starch. In a prescription for suppository, the suppository can be produced by mixing the present inventive VHH antibody with a common suppository base such as cacao butter.

Furthermore, the present inventive VHH antibody can be prescribed as a sustained-release preparation by encapsulation in a polymer matrix or the like.

The pharmaceutical composition of the present invention is useful as a pharmaceutical composition for increasing the peptide antimicrobial susceptibility of *Pseudomonas aeruginosa* and is therefore preferably used in combination with a peptide antimicrobial agent. Examples of the peptide antimicrobial agent include colistin or its salt and polymyxin B.

Among them, colistin or its salt is preferable, and colistin or colistin methanesulfonate is more preferable.

Colistin is thought to be a "trump" to multidrug-resistant gram-negative bacilli, but is highly nephrotoxic and has a narrow efficacy and safety margins. It is inferred that a combination use with the present inventive VHH antibody can decrease the dose of colistin and leads to safer therapeutic strategy.

In the combination use of the pharmaceutical composition of the present invention and a peptide antimicrobial agent, these drugs may be administered simultaneously, or one may be administered first followed by the other.

### Examples

The present invention will now be described in more detail with reference to Examples, but is not limited to these Examples.

### [Method]

### (1) Production of antigen

Peptides that are each composed of 10 amino acids of the extracellularly protruding loop 1 or loop 2 of OprM, sequences of 5 amino acids added to before and after the 10 amino acids, and cysteine added to the C-terminal thereof were synthesized, and thyroglobulin and Keyhole limpet hemocyanin (KLH) were added as carrier proteins to the loop 1 peptide and the loop 2 peptide, respectively, to obtain antigens (Fig. 2).

### (2) Immunization of alpaca

An emulsion prepared by mixing 500 µg/mL of an antigen (loop 1 peptide-thyroglobulin or loop 2 peptide-KLH) and an equal amount of adjuvant (Titer Max for the first time and Freund's adjuvant thereafter) was dispersively administered at 10 sites in the thigh muscles of a 5-year-old female alpaca. The immunization was carried out 6 times in total at one-week intervals. Ten milliliter of blood was collected before immunization, 5 weeks after immunization, and 7 weeks after immunization. Thirty milliliter of blood was collected 10 weeks after immunization with the loop 1 antigen and was collected 14 weeks after immunization with the loop 2 antigen.

### (3) Antibody titer measurement

Loop 1 peptide-ovalbumin (5 µg/mL) or loop 2 peptide (5 µg/mL) was dispensed in a 96-well plate at 50 µL per well and was immobilized. Block Ace solution was added thereto for blocking, and 100 µL of serially diluted serum samples were then added thereto, followed by reaction for 1 hour. The wells were then washed, and 100 µL of 10 000-times diluted anti-llama goat IgG-HRP (Bethyl Laboratories, Inc.) was added to each well as a secondary antibody, followed by reaction for 1 hour. The wells were washed, and ABTS and H₂O₂ substrate solution (Thermo Fisher Scientific) were applied thereto for 15 minutes. Absorbance at 415 nm was measured with a microplate reader (Corona Electric Co., Ltd.).

### (4) Monocyte preparation, RNA extraction, and cDNA synthesis

Heparin-treated blood was added to Leucosep (Greiner Bio-One) filled with Lymphoprep (VERITAS Corporation) and centrifugated at 20°C at 800 × g for 30 minutes, and a fraction containing monocytes was collected. RNAlater (QIAGEN) was added thereto to obtain suspension, which was then cryopreserved at -80°C. The frozen samples were shaken and thawed at 37°C, and Buffer RLT was added thereto. The mixture was applied to QIAshredder spin column (QIAGEN) and centrifugated at 18 000 × g for 2 minutes, and RNA was extracted from monocytes using RNeasy Mini Kit (QIAGEN). Single strand cDNA was synthesized with PrimeScript II 1^{st} strand cDNA synthesis kit (Takara Bio Inc.) using Oligo dT primer and Random 6mer primer (about 2.5 µg of total RNA was used for 20 µL of reaction solution).

### (5) Alpaca library production

A gene of a VHH region was amplified from cDNA by PCR. The VHH antibody gene was amplified using PrimeSTAR GXL DNA polymerase (Takara Bio Inc.) by PCR at 95°C for 1 minute: 1 cycle, and 98°C for 10 seconds, 55°C for 15 seconds, and 68°C for 1 minute: 25 cycles (0.5 µL of cDNA reaction solution was used for 25 µL of PCR reaction solution).

PCR of the 1^{st} PCR product was similarly performed at 95°C for 1 minute: 1 cycle, and 98°C for 10 seconds, 60°C for 15 seconds, and 68°C for 1 minute: 25 cycles to amplify the antibody gene (5 pg to 10 pg of the 1^{st} PCR product was used for 25 µL of PCR reaction solution). The primer DNA sequences for PCR were as follows:
F: 5'-GGTGGTCCTGGCTGCTCTT-3' (SEQ ID No. 11)
R: 5'-AGGAGACGGTGACCTGGGT-3' (SEQ ID No. 12).
   Primers for 2nd PCR: 
F: 5'-GGTAAAGGCCCAGCCGGCCATGGCCGAGGTGCAGCTCGTGGAGTCTGGG-

3' (SEQ ID No. 13)
   SfiI
      R: 5'-TTGCAAGCAATTGCGGCCGCTGAGGAGACGGTGACCTGGGT-3' (SEQ ID No. 14)
   NotI.

The 2^{nd} PCR product (VHH gene) was digested with SfiI and NotI, inserted into the SfiI and NotI sites of pCANTAB6 vector (provided by Professor Yuji Ito, Faculty of Science and Engineering, Kagoshima University), and introduced into *Escherichia coli* TG1 competent cells (Lucigen Corporation) by electroporation, and TG1 was infected with M13K07 helper phage (provided by Professor Yuji Ito, Faculty of Science and Engineering, Kagoshima University) to obtain a phage library expressing VHH region. Amersham Bioscience Expression Module/Recombinant Phage Antibody System instruction manual was referred to.

### (6) Library screening

Biopanning of the produced phage library was performed for selecting a phage expressing a VHH antibody to a target antigen. Thyroglobulin-loop 1 peptide or KLH-loop 2 peptide was immobilized on an immune tube and blocked, and a phage library containing 200 µg/mL of thyroglobulin or KLH was reacted thereto at 4°C overnight, followed by washing with PBS (137 mmol/L of NaCl, 8.1 mmol/L of Na₂HPO₄, 2.68 mmol/L of KCl, 1.47 mmol/L of KH₂PO₄, pH 7.4) and PBS-0.1% (v/v) Tween 20 20 times each to remove phages that did not bind. Two milliliter of 0.1 M Glycine HCl (pH 2.8) was added to the immune tube, and after pipetting, 200 µL of 1 M Tris HCl (pH 9.0) was put in the tube for neutralization, and the phage bound to the tube was eluted.

The eluted phage was re-infected to *Escherichia coli* and amplified. The above procedure was repeated 4 times to concentrate the phage specific to the target antigen. The panned phage was re-infected to *Escherichia coli* TG1, TG1 clone containing VHH-inserted pCANTAB phagemid vector was then isolated, and KO7 helper phage was infected to individual TG1 clones to obtain cloned phages displaying VHH antibodies. From these cloned phages, clones that react with loop 1 or loop 2 antigen were respectively selected to determine the sequences of the antibody genes.

### (7) Verification of binding of VHH antibody monomer to loop 1 or loop 2 antigen by ELISA

In the anti-loop 1 VHH antibody, 0.1 M NaHCO₃ (Blank), 0.1 M NaHCO₃ containing 10 µg/mL of thyroglobulin, 0.1 M NaHCO₃ containing 10 µg/mL of thyroglobulin-loop 1 peptide, or 0.1 M NaHCO₃ containing 10 µg/mL of ovalbumin-loop 1 peptide was added to a 96-well immuno plate (Nunc) at 40 µL/well and was left to stand at 4°C overnight.

In the anti-loop 2 VHH antibody, 0.1 M NaHCO₃ (Blank), 0.1 M NaHCO₃ containing 10 µg/mL of KLH, or 0.1 M NaHCO₃ containing 10 µg/mL of KLH-loop 2 peptide was added to a 96-well immuno plate (Nunc) at 40 µL/well and was left to stand at 4°C overnight. SuperBlock-PBS (Thermo Fisher Scientific) was added to the plate at 350 µL/well and left to stand at room temperature for 1 hour. After washing with 400 µL/well of PBS-T (phosphate buffer saline containing 0.05% Tween 20), 40 µL/well of SuperBlock-TBS containing 0.2 µg/mL of the VHH antibody was added thereto, followed by reaction at room temperature for 1 hour. The wells were washed with 400 µL/well of PBS-T again three times, and 40 µL/well of anti-Myc mouse monoclonal antibody 9E10 (Santa Cruz Biotechnology Inc.) diluted 500 times with SuperBlock-TBS was added thereto, followed by reaction at room temperature for 1 hour. After washing with 400 µL/well of PBS-T three times, 40 µL/well of anti-mouse IgG goat antibody HRP was added thereto, followed by reaction at room temperature for 1 hour. Subsequently, the wells were washed with 400 µL/well of PBS-T three times, 50 µL/well of TMB reagent (FUJIFILM Wako Pure Chemical Corporation) was added thereto, reaction was performed for about 10 minutes, and the reaction was stopped with 0.5 M sulfuric acid. Subsequently, absorbance at 450 nm was measured, and the average and standard deviation were calculated of triplicate or quadruplicate measurement.

### (8) Expression and purification of recombinant 4P94 monomer protein or 4P41 monomer protein in E. coli BL21 (DE3)

Genes for *Escherichia coli* expression were DNA-synthesized based on the amino acid sequence information on 4P94 or 4P41. An NdeI-NotI DNA fragment was inserted into the NdeI and NotI sites of pET22b(+) (Novagen) to construct a gene encoding a protein in which cMyc-Tag and His-Tag were added to the C-terminal side of a VHH antibody. An *E. coli* RosettaGami^{™} 2 (DE3) competent cell (Novagen) was transformed with pET22b(+) plasmid DNA into which the VHH antibody DNA fragment had been inserted. The recombinant *Escherichia coli* was cultured at 37°C using 200 mL of a 2YT medium containing 100 µg/mL of ampicillin (Sigma-Aldrich), and 1 mM IPTG (isopropyl-β-thiogalactopyranoside, Takara Bio Inc.) was added thereto at the time when OD₆₀₀ reached 0.4 to 0.5, followed by culturing at 30°C for 3 hours. After completion of the culturing, *Escherichia coli* was collected and resuspended in 20 mL of extraction buffer (50 mM Na Phosphate, pH 7.8, 300 mM NaCl, EDTA-Free protease inhibitor cocktail (Roche Diagnostics)). Ultrasonication treatment was performed in ice using Sonifier 250 (Branson Ultrasonics) twice for 1 minute under conditions of Output Control 2 and Duty cycle 80% to disrupt the bacterial cells. The suspension after the treatment was centrifugated at 20 000 ×g at 4°C for 20 minutes, and the supernatant was collected. The protein was purified using a His-Trap column (GE Healthcare). The centrifuge supernatant of ultrasonicated suspension was directly applied to a His-Trap column, the column was washed with a binding buffer (20 mM sodium phosphate, 0.5 M NaCl, 20 mM imidazole, pH 7.4), and the fusion protein was then eluted with an eluate containing 500 mM imidazole. The purified sample was subjected to SDS electrophoresis and then stained with TaKaRa CBB Protein Safe Stain (Takara Bio Inc.) to confirm 4P94 or 4P41 monomer protein at about 20 kDa. In addition, western blotting was performed using anti-His-Tag mouse monoclonal antibody HRP.

### (9) 4P41 dimer and 4P41 trimer

A gene encoding a 4P41 dimeric antibody or 4P41 trimeric antibody in which NdeI site, Strep Tag²⁵, two or three 4P41 monomer sequences linked with a linker peptide (GGSG), HindIII site, Myc-Tag, and NotI site were bound was chemically synthesized.

### (10)4P94 trimer

A gene encoding a 4P94 trimeric antibody in which NdeI site, Strep Tag, three 4P94 monomer sequences linked with a linker peptide (GGSG), HindIII site, Myc-Tag, and NotI site were bound was chemically synthesized.

### (11) 4P94 LZ trimer and 4P41 LZ trimer

A gene encoding a 4P94 LZ trimer or 4P41 LZ trimer in which NdeI site, a 4P94 monomer sequence or 4P41 monomer sequence, HindIII site, a (GGSG) linker, a trimer-forming leucine zipper motif, Myc-Tag, and NotI site were bound was chemically synthesized.

An NdeI-NotI DNA fragment was inserted into the NdeI and NotI sites of pET22b(+) (Novagen) to construct a gene encoding a protein in which cMyc-Tag and His-Tag were added to the C-terminal side of a VHH antibody. An *E*. *coli* RosettaGami 2 (DE3) competent cell (Novagen) was transformed with pET22b(+) plasmid DNA into which the VHH antibody DNA fragment had been inserted. The recombinant *Escherichia coli* was cultured at 37°C using 200 mL of a 2YT medium containing 100 µg/mL of ampicillin (Sigma-Aldrich), and 0.1 mM IPTG (Takara Bio Inc.) was added thereto at the time when OD₆₀₀ reached 0.3, followed by culturing at 20°C for 16 hours. After completion of the culturing, *Escherichia coli* was collected and resuspended in 20 mL of extraction buffer (50 mM Na Phosphate, pH 7.8, 300 mM NaCl, EDTA-Free protease inhibitor cocktail (Roche Diagnostics)). Bacterial cell disruption used Micro Smash (Tomy Seiko Co., Ltd.). Three gram of 0.1 mm diameter zirconia beads (Tomy Seiko Co., Ltd.) was added to each of 16 5-mL sample tubes (Tomy Seiko Co., Ltd.), 5 mL of a bacterial cell suspension was added to each of the tubes, and disruption at 4°C at 4 200 rpm for 5 minutes was repeated three times. After the disruption, centrifugation was performed at 20 000 ×g at 4°C for 15 minutes, and the supernatant was collected and filtered through a 0.45 µm diameter filter (Merck Millipore). Purification of protein was performed using a His-Trap column (Cytiva) for the 4P94 trimer and the 4P94 LZ trimer. The centrifuge supernatant of the bead disruption solution was applied to a His-Trap column, the column was washed with a binding buffer (20 mM sodium phosphate, 0.5 M NaCl, 20 mM imidazole, pH 7.4), and the fusion protein was then eluted with an eluate containing 500 mM imidazole. The 4P94 LZ trimer was purified again by ion exchange chromatography using AKTA pure 25 (Cytiva) as the eluate. The fusion protein was eluted using Resource S (Cytiva) as the column with a salt concentration gradient from 25 mM MES pH 5.6 to 25 mM MES pH 5.6, 0.5 M NaCl. Purification of 4P41 dimer, 4P41 trimer, and 4P41 LZ trimer used Protein A column (Cytiva). The centrifuge supernatant of the bead disruption solution was applied to the Protein A column and washed with a binding buffer (1.5 M glycine/NaOH, pH 9.0, 0.5 M NaCl), and the fusion protein was eluted with an eluate (0.1 M glycine/HCl pH 2.5). The eluted fusion protein was subjected to endotoxin removal treatment using a NoEndo HC spin column kit (Protein Ark), and it was then verified using EndoLISA (J. K. International) that the endotoxin concentration was less than 1 EU/mL. The fusion protein was dialyzed using an extraction buffer (50 mM Na Phosphate, pH 7.8, 300 mM NaCl).

### (12) Measurement of dissociation constant (KD) between 4P41 monomer and KLH-loop 2 peptide

The binding affinity between 4P41 monomer and KLH-loop 2 was analyzed by surface plasmon resonance using Biacore X-100 (Cytiva). The measurement was performed by a Multicycle kinetics method in accordance with the manual attached to Biacore X-100, and 4P41 monomer was measured at 4.4 nM, 89 nM, 178 nM, 355 nM, and 710 nM.

### (13) Gel filtration chromatography of 4P41 LZ trimer

The purification sample of the 4P41 LZ trimer was added to gel filtration column HiLoad 16/60 Superdex 200 pg (Cytiva) equilibrated with SEC Running buffer (50 mM Tris, pH 8.0, 150 mM NaCl) and was separated at a flow rate of 1 mL/min. Liquid delivering and detection used AKTA Prime Plus (Cytiva). The molecular weight was estimated from a calibration curve using globular proteins of known molecular weights as an indicator. The calibration curve was produced using LMW Gel filtration calibration kit (Cytiva).

### (14) Cytotoxicity test of 4P41 LZ trimer

Human liver cancer HepG2 cells (American Type Culture Collection) were suspended in a DMEM medium (Sigma-Aldrich) containing 10% fetal bovine serum (Tissue Culture Biologicals) and were added to a Falcon 96-well culture plate (Becton, Dickinson and Company) at 3 × 10³ cells/50 µL medium/well. After culturing for two nights, 50 µL of a medium containing the 4P41 LZ trimer or an antibody solvent extraction buffer (50 mM Na phosphate, pH 7.8, 300 mM NaCl) was added to each well at a final concentration of 10 µg/mL to 160 µg/mL. After further culturing for two nights, 10 µL Cell counting kit-8 (Dojindo Laboratories) was added thereto. After culturing for 1 hour, absorbance at 450 nm was measured. The well containing only cells was set to 100%, and the relative values thereto were used as data, which are shown as the average ± standard error of 5 wells of each concentration.

### (15) Drug susceptibility test

The minimum growth inhibitory concentration (MIC) of an antimicrobial agent was measured by a microbroth dilution method in accordance with the Clinical Laboratory Standards Institute (CLSI) guideline. The serially diluted antimicrobial agent was added to a 96-well round bottom microtiter plate (Watson Co., Ltd.) at 100 µL/well, and each bacterial strain was inoculated at 5 × 10⁵ CFU/mL/well. As the antimicrobial agent, Aztreonam (Sigma-Aldrich), Amikacin (Sigma-Aldrich), Arbekacin (Meiji Seika Pharma Co., Ltd.), Ofloxacin (Sigma-Aldrich), colistin (Sigma-Aldrich), and Polymyxin B (Sigma-Aldrich) were used. Synergistic effect of an antibody and an antimicrobial agent was evaluated using a checkerboard dilution method. Each of the antimicrobial agents was serially two-fold diluted in the vertical direction in a 96-well round bottom titer plate to concentrations between 0.125 to 2 times the MIC. Subsequently, an antibody was serially two-fold diluted in the horizontal direction of the plate in a range of 0 to 10 µg/mL or 0 to 20 µg/mL. Each bacterial strain was inoculated at 5 × 10⁵ CFU/mL/well.

### (16) Used strain

A multidrug-resistant *Pseudomonas aeruginosa* NCGM2.S1 and drug-susceptible *Pseudomonas aeruginosa* PAO1 and OCR1 were cultured using Luria Bertani (LB) medium (BD) or an LB plate containing 15 g/L of agar at 37°C. OprM-deficient *Pseudomonas aeruginosa* TN0721ΔOprM (provided by Associated Professor Eisaku Yoshihara, Tokai University School of Medicine) was cultured in an LB medium containing 50 µg/mL of Sulbenicillin (MP Biomedicals). Drug-susceptible *Escherichia coli* ATCC25922 and *Staphylococcus aureus* ATCC25923 were cultured using a tryptic soy broth at 37°C. Drug-susceptible *Acinetobacter baumannii* ATCC15308 was cultured using Difco^{™} Nutrient broth (BD) at 37°C.

### (17) Pseudomonas aeruginosa infection experiment with silkworm

Fertilized eggs of silkworm larvae (Shunrei Shogetsu, Ehime silkworm species) were disinfected with 70% ethanol (FUJIFILM Wako Pure Chemical Corporation), 4% formalin (FUJIFILM Wako Pure Chemical Corporation), and 99.5% ethanol (FUJIFILM Wako Pure Chemical Corporation) and were bred with artificial feed Silkmate 2S (Nosan Corporation) in an incubator of 27°C. The silkworm larvae were transferred to a new feeding box one day before shedding to 5th instar, and were fed with artificial feed not containing antibiotics Morus for 2nd instar (Sysmex Corporation) for 1 day. Overnight culture solution of multidrug-resistant *Pseudomonas aeruginosa* NCGM2.S1 was diluted 100 times and was further cultured overnight. The bacterial culture solution was diluted with physiological saline (Otsuka Pharmaceutical Co., Ltd.), and 0.05 mL thereof was injected into hemolymph from the dorsal surface using a 1-mL injection syringe (Terumo Corporation) and 27-gauge syringe needle (Terumo Corporation), and the silkworm larvae were fed at 27°C. The median lethal dose was measured from the survival rate after 24 hours, and 0.05 mL of the bacteria in an amount 4 times the median lethal dose was injected to make a silkworm *Pseudomonas aeruginosa* infection model. To the silkworm *Pseudomonas aeruginosa* infection model, 0.05 mL of colistin or colistin and the 4P41 LZ trimer was injected into hemolymph, and the number of surviving was counted over 4 days after the injection.

### (18) Membrane potential measurement

1 × 10⁸ CFU/mL of multidrug-resistant *Pseudomonas aeruginosa* NCGM2.S1 was washed with PBS, and 1.1 M glucose was added thereto, followed by incubation at 37°C for 15 minutes. 50 µM DiBAC₄ (3) (Dojindo Laboratories) and the 4P94 LZ trimer or the 4P41 LZ trimer or an anti-human TRAIL-R1 VHH antibody 4P6 trimer or PaβN (Med Chemexpress) were added thereto, and further incubation was then performed for 60 minutes, and the absorbance was measured with a microplate reader at an excitation wavelength of 490 nm and a maximum fluorescence wavelength of 516 nm.

### (19) FACS analysis

Regarding *Pseudomonas aeruginosa* NCGM2.S1, TNP0721ΔOprM, *Acinetobacter baumannii* ATCC15308, *Escherichia coli* ATCC25922, and *Staphylococcus aureus* ATCC25923, 1 × 10⁸ CFU/mL of each of the bacteria was reacted in 30 µL of PBS containing 10 µg/mL of the 4P41 LZ trimer or the 4P6 trimer as a negative control on ice for 60 minutes. After washing with PBS, the bacteria were reacted in 30 µL of PBS containing 0.2 µg/mL of anti-His-tag mA (Medical & Biological Laboratories Co., Ltd.) on ice for 60 minutes. After washing with PBS, the bacteria were reacted in 30 µL of PBS containing 3 µg/mL of Alexa Fluor^{™} 488-conjugated AffiniPure Goat Anti-Mouse IgG (Jackson Immuno Research Inc.) on ice for 30 minutes. After washing with PBS, the bacteria were reacted in 30 µL of PBS containing 0.5% paraformaldehyde on ice for 10 minutes. After washing with PBS, analysis with FACSVerse (BD Biosciences) was performed.

### [Results]

### (1) Production of anti-loop 1 and anti-loop 2 VHH antibodies

OprM is composed of 468 amino acids and includes two loops protruding to the outside the cell (Fig. 1 B). Antigens corresponding to the extracellularly protruding loop 1 and loop 2 of OprM were produced as described in the section of Method above and were immunized to alpacas. Antibody titers were measured by ELISA using serum 5 weeks after immunization with the loop 1 antigen and serum 7 weeks after immunization with the loop 2 antigen (Fig. 3). Since an increase in the antibody titer was confirmed, 30 mL of blood was collected 10 weeks after immunization with the loop 1 antigen and 14 weeks after immunization with the loop 2 antigen, and monocyte fractions were prepared. RNAs were extracted from the monocyte fractions, cDNAs were synthesized, and genes of the VHH region were amplified by PCR. The VHH obtained from total RNA extracted from the monocyte fraction of the alpaca immunized with the loop 1 antigen was 1.2 × 10⁵ types, and the VHH obtained from the total RNA extracted from the monocyte fraction of the alpaca immunized with the loop 2 antigen was 6.3 × 10⁵ types. The VHH genes were incorporated into pCANTAB6 and were introduced into *Escherichia coli* TG1 competent cells by electroporation to infect TG1 with M13K07 helper phage. Phage libraries derived from 3 × 10⁷ TG1 colonies were obtained from the respective total RNAs derived from the monocyte fractions immunized with the loop 1 antigen or the loop 2 antigen. Phages expressing VHH antibodies to the loop 1 or loop 2 antigen in the phage libraries were concentrated by biopanning.

The phage pool panned four times had the highest reactivity with the loop 1 or loop 2 peptide (Fig. 4). Phages of anti-loop 1 library and anti-loop 2 library panned four times were re-infected to *Escherichia coli* TG1, TG1 *Escherichia coli* colonies containing VHH-inserted pCANTAB phagemid vector were then isolated, and the TG1 *Escherichia coli* clones were each infected with a K07 helper phage. Screening by ELISA using phage clones displaying an anti-loop 1 VHH antibody and an anti-loop 2 VHH antibody was performed (Fig. 5). Consequently, a plurality of phage clones showed specific binding to each antigen peptide (Fig. 6 A and B).

VHH antibodies are classified into a VHH type [37Phe/Tyr, 44Glu, 45Arg, and 47Gly] and a VH type [37Val, 44Gly, 45Leu, and 47Trp] depending on the FR2 region amino acid sequences. The amino acid sequences of the phage clones that showed specific binding to the loop 1 or loop 2 peptide were analyzed. As a result, 4P94 was obtained as an anti-loop 1 VHH antibody, and 4P41 was obtained as an anti-loop 2 VHH antibody (Fig. 7). Genes for *Escherichia coli* expression were designed based on the amino acid sequence information on 4P94 and 4P41, genes of the 4P94 monomer, 4P94 LZ trimer, 4P41 dimer, 4P41 trimer, and 4P41 LZ trimer were synthesized, and vectors for *Escherichia coli* expression were produced (Fig. 8). The expression vectors were each inserted into pET22b(+) and introduced into *E. coli* RosettaGami^{™} 2 (DE3) (Novagen). IPTG was added to recombinant *Escherichia coli* to express fusion proteins, which were column-purified and were verified by SDS-PAGE (Fig. 9). The purified 4P94 monomer protein specifically bound to the ovalbumin-loop 1 peptide, and the purified 4P41 monomer protein specifically bound to the KLH-loop 2 peptide (Fig. 10 A and B). The binding affinity between the 4P41 monomer and the antigen KLH-loop 2 was analyzed by surface plasmon resonance, and the KD value between the 4P41 monomer and the KLH-loop 2 was 2.841 × 10⁻⁷ M (Fig. 11).

Since the 4P41 LZ trimer is detected as a monomer by SDS-PAGE, in order to verify the formation of a trimer, analysis with a gel filtration column was performed. As a result of the gel filtration column analysis, one monodisperse main peak was observed and the molecular weight estimated from a calibration curve using spherical proteins of known molecular weights as an indicator was 55.5 kDa, which suggested that a trimer was formed (Fig. 12). No peak corresponding to a monomer was observed to suggest all molecular species formed multimers.

### (2) Antimicrobial susceptibility-increasing effect of antibody on multidrug-resistant Pseudomonas aeruginosa

The 4P41 LZ trimer was added to multidrug-resistant *Pseudomonas aeruginosa* strain NCGM2.S1, and changes in the minimum growth inhibitory concentration (MIC) of Aztreonam, Amikacin, Arbekacin, Ofloxacin, colistin, or Polymyxin B were investigated by a microbroth dilution method (Fig. 14 A). In the 4P41 LZ trimer addition group, the MICs of colistin and Polymyxin B decreased to 1/4, but the MICs of Aztreonam, Amikacin, Arbekacin, and Ofloxacin did not change.

The MIC of colistin when the 4P94 monomer, 4P94 LZ trimer, 4P41 monomer, 4P41 dimer, 4P41 trimer, or 4P41 LZ trimer was added at a concentration of 10 µg/mL to multidrug-resistant *Pseudomonas aeruginosa* was measured using a microbroth dilution method (Fig. 14 B). When the 4P94 LZ trimer, 4P41 trimer, or 4P41 LZ trimer was added, the MIC of colistin was decreased to 1/4. When the 4P94 monomer, 4P41 monomer, or 4P41 dimer was added, the MIC of colistin was decreased to 1/4. When the 4P94 LZ and 4P41 LZ were added at 15 µg/mL each, the MIC of colistin was decreased to 1/4. When the 4P6 trimer, which is a human TRAIL-R1 VHH antibody trimer, was added, the MIC of colistin did not change. In both 4P94 and 4P41 antibodies, the activity of increasing the colistin susceptibility of multidrug-resistant *Pseudomonas aeruginosa* was higher in the trimer than in the monomer and dimer.

The changes in the minimum growth inhibitory concentration (MIC) of Aztreonam, Amikacin, Arbekacin, Ofloxacin, colistin, and Polymyxin B when the 4P94 LZ trimer was added to multidrug-resistant *Pseudomonas aeruginosa* strain NCGM2.S1 were investigated by a microbroth dilution method (Fig. 14 C). When the 4P94 LZ trimer was added, the MICs of colistin and Polymyxin B were decreased to 1/4.

### (3) Colistin susceptibility-increasing effect of antibody on each bacterium

The 4P94 LZ trimer or 4P41 LZ trimer was added at 10 µg/mL to wild-type *Pseudomonas aeruginosa* PAO-1 and OCR1, OprM deficiency *Pseudomonas aeruginosa* strain TNP0721ΔOprM, *Acinetobacter baumannii* ATCC15308, *Escherichia coli* ATCC25922, and *Staphylococcus aureus* ATCC25923, the change in the MIC of colistin was investigated by a microbroth dilution method (Fig. 15). When the 4P41 LZ trimer was added, the MIC of colistin in each of *Pseudomonas aeruginosa, Acinetobacter baumannii,* and *Escherichia coli* was decreased to 1/4, but the MIC of colistin in *Staphylococcus aureus* remained unchanged at > 128 µg/mL.

### (4) Cytotoxicity test of 4P41 LZ trimer

The cytotoxicity of the 4P41 LZ trimer on human liver cancer HepG2 cells (American Type Culture Collection) was investigated (Fig. 16). The cell survival rate when the 4P41 LZ trimer was added until 160 µg/mL to HepG2 was the same as the survival rate when an extraction buffer that is the solvent of the antibody was added. It was demonstrated that the 4P41 LZ trimer was nontoxic to human cells.

### (5) Production of silkworm multidrug-resistant Pseudomonas aeruginosa infection model and colistin susceptibility-increasing effect of antibody

NCGM2.S1 culture solution was injected into hemolymph of silkworms, and the concentration that causes 50% death after 24 hours was investigated (Fig. 17). The LD₅₀ of NCGM2.S1 in silkworm calculated by probit analysis was estimated to be 0.4 × 10⁷ CFU/mL. As the concentration that causes 100% death after 24 hours, 7 × 10⁷ CFU/mL of NCGM2.S1 was injected to form a silkworm *Pseudomonas aeruginosa* infection model. Colistin alone or colistin and the 4P41 LZ trimer or 4P94 LZ trimer was administered to the silkworm *Pseudomonas aeruginosa* infection model, and the therapeutically effective doses ED₅₀ of colistin in each group were compared (Fig. 18). The ED₅₀ of colistin in each group was calculated by probit analysis. As a result, the ED₅₀ was estimated to be 124 µg/g body weight in the colistin alone administration group, 71 µg/g body weight in the colistin and 4P41 LZ trimer administration group, and 85 µg/g body weight in the colistin and 4P94 LZ trimer administration group. The therapeutically effective doses of colistin in the 4P41 LZ trimer administration group and the 4P94 LZ trimer administration group were decreased compared to the colistin alone administration group, and it was demonstrated that the colistin susceptibility-increasing effects of the 4P41 LZ trimer and 4P94 LZ trimer were recognized in vivo. In addition, the 4P41 LZ trimer and the 4P94 LZ trimer did not show toxicity to silkworm even in administration of 22 µg per body weight (Fig. 19 A and B). In the silkworm *Pseudomonas aeruginosa* infection model, since the therapeutically effective dose of colistin was decreased by administration of 1.2 to 2.4 µg per body weight of the 4P41 LZ trimer or 4P94 LZ trimer, no toxicity to silkworm was observed even when 9 to 18 times amount of the antibody was administered.

### (6) FACS analysis of 4P41 LZ trimer and each bacterium

Since the colistin susceptibility-increasing effect of the 4P41 LZ trimer was observed in OprM deficiency *Pseudomonas aeruginosa* strain, *Acinetobacter baumannii,* and also *Escherichia coli,* it was confirmed by FACS analysis that the 4P41 LZ trimer binds to these bacteria (Fig. 20). It was confirmed that multidrug-resistant *Pseudomonas aeruginosa* NCGM2.S1, OprM deficient *Pseudomonas aeruginosa* strain TNP0721ΔOprM, *Acinetobacter baumannii* ATCC15308, and *Escherichia coli* ATCC25922 bind to the 4P41 LZ trimer. *Staphylococcus aureus* whose susceptibility to colistin did not change by adding the 4P41 LZ trimer did not bind to the 4P41 LZ trimer. In homology search by BLAST, *Escherichia coli* and *Acinetobacter baumannii* included sequences that are respectively 60% and 100% identical to the loop 1 antigen sequence and included sequences that are respectively 74% and 100% identical to the loop 2 antigen sequence. In addition, OprJ, which is an OprM subfamily protein, included a sequence that is 40% identical to the loop 1 antigen sequence and a sequence that is 60% identical to the loop 2 sequence, OpmA included a sequence that is 40% identical to the loop 2 antigen sequence, and OpmB included a sequence that is 40% identical to the loop 1 sequence. Accordingly, a possibility that the antibody binds to an efflux pump other than OprM was suggested.

In addition, the TolC family protein of *Pseudomonas aeruginosa* is 100% identical to the loop 2 antigen sequence and therefore has a possibility of binding to the antibody.

### (7) Influence of antibody on membrane potential of Pseudomonas aeruginosa

Colistin and Polymyxin B whose susceptibility of *Pseudomonas aeruginosa* was increased by an antibody shows a positive charge and binds to the outer membrane of a bacterium to collapse the cross-linked structure of calcium and magnesium to leak cell contents, leading to cell death. Influence of an antibody on the bacterial outer membrane was investigated using a membrane potential-sensitive dye (Fig. 21), and when the 4P94 LZ trimer and the 4P41 LZ trimer were added, the potential of the *Pseudomonas aeruginosa* outer membrane changed antibody dose-dependently. A possibility was suggested to enhance the effect of colistin by destabilizing the *Pseudomonas aeruginosa* outer membrane by an antibody.

### (8) Humanization of 4P94 and 4P41 antibodies

Humanization of 4P94 and 4P41 was investigated. The amino acid sequence of a humanized antibody was designed based on the humanization model of the VHH antibody that binds to cell death receptor DR5 (Fig. 22), and a humanized 4P41LZ trimer was produced.

Expression and purification of recombinant humanized 4P41LZ trimer protein in *E. coli* BL21 (DE3)
1. A gene encoding a humanized 4P41 VHH LZ trimer in which NdeI site, a humanized 4P41 VHH monomer sequence, a (GGSG) linker, a trimer-forming leucine zipper motif, Myc-Tag, and NotI site were bound was chemically synthesized.
2. An NdeI-NotI DNA fragment was inserted into the NdeI and NotI sites of pET22b(+) (Novagen) to construct a gene encoding a protein in which cMyc-Tag and His-Tag were added to the C-terminal side of a VHH antibody. An *E*. *coli* RosettaGami 2 (DE3) competent cell (Novagen) was transformed with pET22b(+) plasmid DNA into which the VHH antibody DNA fragment had been inserted.
3. The recombinant *Escherichia coli* was cultured at 37°C using 200 mL of a 2YT medium containing 100 µg/mL of ampicillin (Sigma-Aldrich), and 0.1 mM IPTG (Takara Bio Inc.) was added thereto at the time when OD₆₀₀ reached 0.3, followed by culturing at 30°C for 3 hours. After completion of the culturing, *Escherichia coli* was collected and resuspended in 20 mL of extraction buffer (50 mM Na Phosphate, pH 7.8, 300 mM NaCl, EDTA-Free protease inhibitor cocktail (Roche Diagnostics)). Bacterial cell disruption used Micro Smash (Tomy Seiko Co., Ltd.). Three gram of 0.1 mm diameter zirconia beads (Tomy Seiko Co., Ltd.) was added to each of 16 5-mL sample tubes (Tomy Seiko Co., Ltd.), 5 mL of a bacterial cell suspension was added to each of the tubes, and disruption at 4°C at 4 200 rpm for 5 minutes was repeated three times. After the disruption, centrifugation was performed at 20 000 ×g at 4°C for 15 minutes, and the supernatant was collected and filtered through a 0.45 µm diameter filter (Merck Millipore).
4. Purification of protein was performed using a His-Trap column (Cytiva). The filtrate of centrifuge supernatant of the bead disruption solution was applied to a His-Trap column, the column was washed with a binding buffer (20 mM sodium phosphate, 0.5 M NaCl, 20 mM imidazole, pH 7.4), and the fusion protein was then eluted with an eluate containing 500 mM imidazole.
5. The purified sample was subjected to 15% acrylamide SDS electrophoresis and then stained with TaKaRa CBB Protein Safe Stain (Takara Bio Inc.). In addition, western blotting was performed using anti-His-Tag mouse monoclonal antibody HRP (Thermo Fisher Scientific).

As a result, in the His-Tag column elution sample, a protein that reacts with an anti-His-Tag mouse monoclonal antibody was recognized at the position of a predicted molecular weight (about 21 kDa) of a humanized 4P41 LZ monomer protein.

## Claims

1. A VHH antibody or a dimer or trimer thereof that recognizes loop 1 or loop 2 in OprM of an RND-type multidrug efflux pump of *Pseudomonas aeruginosa.*

2. The VHH antibody or a dimer or trimer thereof according to Claim 1, wherein CDR1 of the VHH antibody that recognizes the loop 1 has an amino acid sequence set forth in SEQ ID NO: 1 or an amino acid sequence having at least 90% homology to the amino acid sequence set forth in SEQ ID NO: 1, CDR2 of the VHH antibody has an amino acid sequence set forth in SEQ ID NO: 2 or an amino acid sequence having at least 90% homology to the amino acid sequence set forth in SEQ ID NO: 2, and CDR3 of the VHH antibody has an amino acid sequence set forth in SEQ ID NO: 3 or an amino acid sequence having at least 90% homology to the amino acid sequence set forth in SEQ ID NO: 3.

3. The VHH antibody or a dimer or trimer thereof according to Claim 1, wherein CDR1 of the VHH antibody that recognizes the loop 2 has an amino acid sequence set forth in SEQ ID NO: 5 or an amino acid sequence having at least 90% homology to the amino acid sequence set forth in SEQ ID NO: 5, CDR2 of the VHH antibody has an amino acid sequence set forth in SEQ ID NO: 6 or an amino acid sequence having at least 90% homology to the amino acid sequence set forth in SEQ ID NO: 6, and CDR3 of the VHH antibody has an amino acid sequence set forth in SEQ ID NO: 7 or an amino acid sequence having at least 90% homology to the amino acid sequence set forth in SEQ ID NO: 7.

4. The VHH antibody or a dimer or trimer thereof according to Claim 1 or 2, wherein the VHH antibody that recognizes the loop 1 has an amino acid sequence set forth in SEQ ID NO: 4 or an amino acid sequence having at least 90% homology to the amino acid sequence set forth in SEQ ID NO: 4.

5. The VHH antibody or a dimer or trimer thereof according to Claim 1 or 3, wherein the VHH antibody that recognizes the loop 2 has an amino acid sequence set forth in SEQ ID NO: 8 or an amino acid sequence having at least 90% homology to the amino acid sequence set forth in SEQ ID NO: 8.

6. The VHH antibody or a dimer or trimer thereof according to any one of Claims 1 to 5, wherein the VHH antibody is a humanized antibody.

7. The humanized VHH antibody or a dimer or trimer thereof according to Claim 6, wherein the humanized VHH antibody that recognizes the loop 1 has an amino acid sequence set forth in SEQ ID NO: 9 or an amino acid sequence having at least 90% homology to the amino acid sequence set forth in SEQ ID NO: 9.

8. The humanized VHH antibody or a dimer or trimer thereof according to Claim 6, wherein the humanized VHH antibody that recognizes the loop 2 has an amino acid sequence set forth in SEQ ID NO: 10 or an amino acid sequence having at least 90% homology to the amino acid sequence set forth in SEQ ID NO: 10.

9. A pharmaceutical composition comprising the VHH antibody or a dimer or trimer thereof according to any one of Claims 1 to 8.

10. The pharmaceutical composition according to Claim 9, wherein the pharmaceutical composition increases peptide antimicrobial susceptibility of *Pseudomonas aeruginosa.*

11. The pharmaceutical composition according to Claim 9 or 10, wherein the pharmaceutical composition is used in combination with a peptide antimicrobial agent.

12. The pharmaceutical composition according to Claim 10 or 11, wherein the peptide antimicrobial agent is colistin.

13. A *Pseudomonas aeruginosa* outer membrane-destabilizing agent comprising the VHH antibody or a dimer or trimer thereof according to any one of Claims 1 to 8 as an active ingredient.

14. The VHH antibody or a dimer or trimer thereof according to any one of Claims 1 to 8, being used for increasing peptide antimicrobial susceptibility of *Pseudomonas aeruginosa.*

15. Use of the VHH antibody or a dimer or trimer thereof according to any one of Claims 1 to 8 for manufacturing a medicament for increasing peptide antimicrobial susceptibility of *Pseudomonas aeruginosa.*

16. A method for increasing peptide antimicrobial susceptibility of *Pseudomonas aeruginosa,* comprising administering the VHH antibody or a dimer or trimer thereof according to any one of Claims 1 to 8.
